# EUROPEAN PATENT APPLICATION

(11) **EP 3 315 595 A1**
(43) Date of publication of application: **02.05.2018**
(21) Application number: 16814127.3
(22) Date of filing: 02.06.2016
(51) Int. Cl.: C12M 1/34

(54) **DETERMINATION DEVICE, DETERMINATION PROGRAM, DETERMINATION METHOD, CELL SHEET MANUFACTURING DEVICE, AND CELL SHEET MANUFACTURING METHOD**

(30) Priority: 26.06.2015 JP 2015128889
(71) Applicant: Nikon Corporation, Tokyo 108-6290 (JP); Tokyo Women's Medical University, Tokyo 162-8666 (JP)
(72) Inventor: UMEZAKI Tadashi, Tokyo 108-6290 (JP); FUKUTAKE Naoki, Tokyo 108-6290 (JP); WATANABE Shunji, Tokyo 108-6290 (JP); TAKI Yusuke, Tokyo 108-6290 (JP); HARAGUCHI Yuji, Tokyo 162-8666 (JP); SHIMIZU Tatsuya, Tokyo 162-8666 (JP)
(74) Representative: Hoffmann Eitle
(86) International application number: PCT/JP2016/066510
(87) International publication number: WO 2016/208356

(57) **Abstract**

Provided is a determination device including a determining section that determines a state of a cell, using information relating to uniformity of a detection target generated based on an optical intensity of radiation light from the detection target included in a biological cell irradiated with excitation light. In the determination device, the detection target may be proteins. The determination device may include an information generating section that generated information, and the information generating section may generate information that excludes information corresponding to a non-resonant background signal.

## Description

### BACKGROUND

### 1. TECHNICAL FIELD

The present invention relates to a determination device, a determination program, a determination method, a cell sheet manufacturing device, and a cell sheet manufacturing method.

### 2. RELATED ART

There is a method for non-invasively evaluating a biological specimen with CARS light (coherent anti-Stokes Raman scattered light) or the like generated by a nonlinear optical effect, as shown in Patent Document 1, for example.
Patent Document 1: Japanese Patent Application Publication No. 2005-062155

The biological specimen is observed by a user to determine a state concerning whether the specimen is alive or dead, and therefore improved work efficiency is desired.

### SUMMARY

According to a first aspect of the present invention, provided is a determination device comprising a determining section that determines a state of a cell, using information relating to uniformity of a detection target generated based on an optical intensity of radiation light from the detection target included in a biological cell irradiated with excitation light.

According to a second aspect of the present invention, provided is a determination program that causes a processor to determine a state of a cell using information relating to uniformity of a detection target generated based on an optical intensity of radiation light from the detection target included in a biological cell irradiated with excitation light.

According to a third aspect of the present invention, provided is a determination method comprising determining a state of a cell using information relating to uniformity of a detection target generated based on an optical intensity of radiation light from the detection target included in a biological cell irradiated with excitation light.

According to a fourth aspect of the present invention, provided is a cell sheet manufacturing device comprising a preparing section that prepares a cell line by isolating a cell; a culturing section that cultures the cell line in a cell sheet; and the determination device described above.

According to a fifth aspect of the present invention, provided is a cell sheet manufacturing method comprising preparing a cell line by isolating a cell; culturing the cell line in a cell sheet; and determining a state of the cell using the determination device described above.

The summary clause does not necessarily describe all necessary features of the embodiments of the present invention. The present invention may also be a subcombination of the features described above.

### BRIEF DESCRIPTION OF THE DRAWINGS

Fig. 1 is a schematic view of the determination device 100.
Fig. 2 is a schematic view showing the basics of a CARS process.
Fig. 3 is a block diagram of the determination device 100.
Fig. 4 is a flow chart showing a determination procedure in the determination device 100.
Fig. 5 shows an image formed by CARS light emitted from proteins.
Fig. 6 shows an image formed by CARS light emitted from lipids.
Fig. 7 shows a combined image.
Fig. 8 shows a state obtained by extracting a region of the cell nucleus from the image.
Fig. 9 is an enlarged view of one cell nucleus in the image.
Fig. 10 shows an image formed by CARS light emitted from proteins.
Fig. 11 shows an image formed by CARS light emitted from lipids.
Fig. 12 shows a combined image.
Fig. 13 shows a state obtained by extracting a region of the cell nucleus from the image.
Fig. 14 is an enlarged view of one cell nucleus in the image.
Fig. 15 is a schematic view of a distribution of contrasts of the luminance values.
Fig. 16 is a schematic view of a distribution of contrasts of the luminance values.
Fig. 17 is a graph showing the occurrence frequency of the luminance values.
Fig. 18 is a graph showing the occurrence frequency of the luminance values.
Fig. 19 is a graph showing a state obtained by shifting the average value to the origin.
Fig. 20 is a graph showing the basics of the uniformity based on Gaussian fitting.
Fig. 21 is a graph showing the basics of the uniformity based on Gaussian fitting.
Fig. 22 is a graph showing the basics of the uniformity based on a cumulative luminance value.
Fig. 23 is a graph showing the basics of the uniformity based on a cumulative luminance value.
Fig. 24 shows an exemplary protein distribution in a living cell.
Fig. 25 is a drawing for describing the basics of the information relating to the uniformity.
Fig. 26 shows an exemplary protein distribution in a dead cell.
Fig. 27 is a drawing for describing the basics of the information relating to the uniformity.
Fig. 28 is a drawing for describing the method for determining the living/dead state of a cell.
Fig. 29 is a drawing for describing the basics of the information relating to the uniformity.
Fig. 30 is a drawing for describing the basics of the information relating to the uniformity.
Fig. 31 is a block diagram of the cell sheet manufacturing device 200.
Fig. 32 is a flow chart showing a procedure for manufacturing a cell sheet in the manufacturing device 200.
Fig. 33 is a schematic cross-sectional view of a structure of the sample 101.
Fig. 34 is an optical micrograph 401 of a reference sample.
Fig. 35 is an optical micrograph 402 of a reference sample.
Fig. 36 schematically shows a process of observing a living cell based on the CARS process.
Fig. 37 schematically shows a process of observing a dead cell based on the CARS process.

### DESCRIPTION OF EXEMPLARY EMBODIMENTS

Hereinafter, some embodiments of the present invention will be described. The embodiments do not limit the invention according to the claims, and all the combinations of the features described in the embodiments are not necessarily essential to means provided by aspects of the invention.

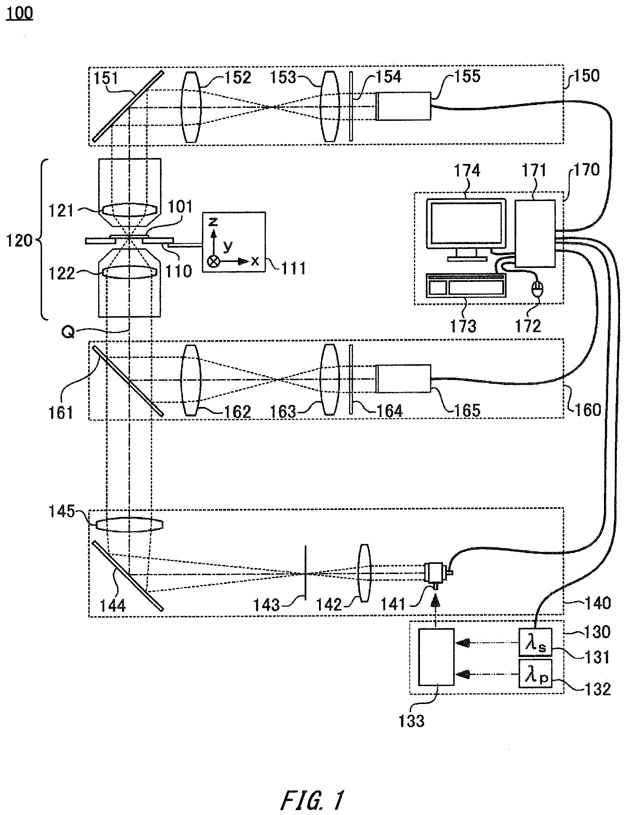

Fig. 1 is a schematic view of an overall configuration of a determination device 100. The determination device 100 includes a stage 110, an objective optical system 120, a laser device 130, an exciting section 140, an upper Raman scattered light detecting section 150, a lower Raman scattered light detecting section 160, and a control section 170.

The stage 110 supports a sample 101 that is a target for the determination by the determination device 100, at an edge portion of a container housing the sample 101. The stage 110 has an opening that exposes the bottom surface of the sample 101 in the drawing. In this way, the sample 101 placed on the stage 110 can be irradiated with excitation light from below in the drawing. Furthermore, radiation light generated from the sample 101 can be detected from below the stage 110.

The stage 110 is coupled to a stage scanner 111. The stage scanner 111 drives the stage 110 both parallel to and perpendicular to the surface on which the sample 101 is placed, as shown by the x, y, and z arrows in the drawing. In this way, in the determination device 100, it is possible to detect the radiation light generated by the sample 101 from a detection target region having a stereoscopic width in the sample 101, while keeping the optical axis of the optical system fixed.

The objective optical system 120 includes an upper objective lens 121 and a lower objective lens 122 that are respectively arranged on opposite sides of the stage 110. In the determination device 100 shown in the drawing, the lower objective lens 122 also serves the role of focusing the excitation light irradiating the sample 101 inside the sample 101.

In the objective optical system 120, the upper objective lens 121 and the lower objective lens 122 preferably have substantially the same number of openings as each other. In this way, when coherent radiation light generated from the sample 101 as a result of irradiation with coherent excitation light is detected, it is possible to prevent a reduction in the detection accuracy due to images or spectral artifacts being superimposed. More specifically, the ratio R_{NA} between the number of openings of the upper objective lens 121 and the number of openings of the lower objective lens 122 preferably satisfies the expression 0.8 < R_{NA} < 1.2.

The laser device 130 includes a plurality of laser light sources 131 and 132 and a combiner 133. The pulse lasers generated by the laser light sources 131 and 132 respectively have wavelengths of λ_{S} and λ_{P} that are different from each other. Mode-locked picosecond Nd:YVO₄ lasers, mode-locked picosecond ytterbium lasers, or the like can be used as the laser light sources 131 and 132, for example.

One of the laser light sources 131 and 132 may be replaced with an optical parametric oscillator that transforms the wavelength of the pulse laser generated by the other laser light source 131 or 132. In order to reduce the invasion into the cells that are the determination target, the pulse length of the pulse laser serving as the excitation light is preferably shorter.

Among the picosecond pulses generated by the laser light sources 131 and 132, the pulse laser with the shorter wavelength λ_{P} can be used as pump light when CARS light, which is one example of Raman scattered light, is generated in the sample 101, for example. Among the picosecond pulses generated by the laser light sources 131 and 132, the pulse laser with the longer wavelength λ_{S} can be used as the excitation light that excites the sample 101, e.g. as Stokes light when generating CARS light. In order to reduce the invasion into the cells that are the determination target, the wavelength of the excitation light is preferably longer.

The laser lights emitted from the laser light sources 131 and 132 are input to the combiner 133 to form a single laser beam. In this way, the excitation light formed by combining the pump light and the Stokes light irradiates the sample 101 at the same position and the same time, thereby making it possible to create a nonlinear optical effect causing Raman scattered light such as CARS light to be generated in the sample 101.

The laser device 130 may include a delayed optical path that delays either one of the pump light or the Stokes light generated by the laser light sources 131 and 132, in order to synchronize the phases of these lights. The delayed optical path can be formed by a plurality of reflective mirrors with changeable intervals therebetween. In the laser device 130, photonic crystal fiber may be used to increase the bandwidth of the Stokes light.

The exciting section 140 includes a galvanic scanner 141 and a scanner lens 142. The galvanic scanner 141 includes a reflective mirror that swings around two swing axes that are not parallel to each other, and two-dimensionally displaces the optical path to which the excitation light is incident in a direction intersecting the optical axis.

The scanner lens 142 focuses the excitation light emitted from the galvanic scanner 141 onto a predetermined primary image surface 143. In this way, the detection target of the sample 101 can be scanned with the excitation light emitted from the laser device 130, and a detection target region having a predetermined width can be irradiated with the excitation light.

The upper Raman scattered light detecting section 150 includes a reflective mirror 151, relay lenses 152 and 153, a band-pass filter 154, and a photoelectric intensifier tube 155. The reflective mirror 151 reflects the Raman scattered light generated in the sample 101 and guides this light to the relay lenses 152 and 153, the band-pass filter 154, and the photoelectric intensifier tube 155. Instead of the reflective mirror 151, a dichroic mirror may be provided that selectively reflects a wavelength of the Raman scattered light.

Furthermore, the band-pass filter 154 transparently passes the Raman scattered light generated from the sample 101 while absorbing or reflecting the excitation light from the exciting section 140. In this way, the photoelectric intensifier tube 155 can efficiently detect the Raman scattered light generated from the sample 101.

The lower Raman scattered light detecting section 160 includes a dichroic mirror 161, relay lenses 162 and 163, a band-pass filter 164, and a photoelectric intensifier tube 165. The dichroic mirror 161 transparently passes, with high efficiency, the excitation light radiated by the exciting section 140 toward the sample 101. Furthermore, the dichroic mirror 161 reflects the Raman scattered light generated in the sample 101 and guides this light to the relay lenses 162 and 163.

The band-pass filter 164 transparently passes the Raman scattered light generated from the sample 101 while absorbing or reflecting the excitation light from the exciting section 140. In this way, the photoelectric intensifier tube 165 can efficiently detect the Raman scattered light generated from the sample 101.

The control section 170 includes a processing device 171, a mouse 172, a keyboard 173, and a display section 174. The mouse 172 and the keyboard 173 are connected to the processing device 171 and are manipulated when inputting instructions from a user to the processing device 171. The display section 174 provides feedback in response to the manipulations of the user performed through the mouse 172 and the keyboard 173, and also displays images or character sequences generated by the processing device 171 to the user. Furthermore, in the determination device shown in the drawings, the determination results are also displayed in the display section 174.

The processing device 171 controls the operations of the laser device 130, the stage scanner 111, and the galvanic scanner 141. Furthermore, the processing device 171 detects the optical intensity of the Raman scattered light, such as the CARS light, generated in the sample 101, and determines the state of the sample 101, e.g. a state concerning whether cells in the sample 101 are living or dead. In this way, it is possible to automate the determination of the living/dead state of the sample 101. The optical intensity can be detected as the luminance, illuminance, luminous intensity, or the like, according to the type of detector used.

With the determination device 100 such as described above, as shown by the dotted lines in the drawing, the excitation light emitted from the exciting section 140 is reflected by the reflective mirror 144 and transparently passed by the dichroic mirror 161, and then irradiates the sample 101 on the stage 110 from below in the drawing. The excitation light is focused within the sample 101 by the lower objective lens 122, and therefore Raman scattered light with a wavelength corresponding to the composition of molecules contained in the sample 101 is generated with a high probability in the sample 101 at the position where the excitation light is focused. Accordingly, with the upper Raman scattered light detecting section 150 and the lower Raman scattered light detecting section 160, it is possible to detect the Raman scattered light generated in the sample 101.

The Raman scattered light detected by the lower Raman scattered light detecting section 160 arranged on the same side of the sample 101 as the exciting section 140 is so-called reflected Raman scattered light that has essentially been reflected by the sample 101. On the other hand, the Raman scattered light detected by the upper Raman scattered light detecting section 150 arranged on the opposite side of the sample 101 from the exciting section 140 is so-called passed Raman scattered light that has essentially transparently passed through the sample 101.

In most cases, the resolution of the detection based on the reflected Raman scattered light is higher than the resolution of the detection based on the passed Raman scattered light. In other words, the detection based on the passed Raman scattered light is suitable for learning a global state of the sample 101. Accordingly, depending on the objective, one of the upper Raman scattered light detecting section 150 and the lower Raman scattered light detecting section 160 is preferably chosen to be used.

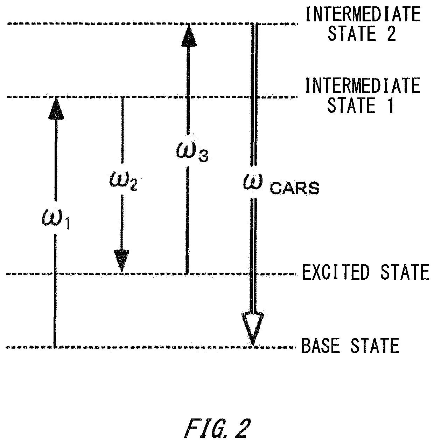

Fig. 2 is a schematic view for describing a CARS process for generating the CARS light, which is an example of Raman scattered light, when the sample 101 is irradiated with the excitation light by the determination device 100. The CARS process occurs when the sample 101 is irradiated with the excitation light including two laser lights, i.e. the pump light and the Stokes light, having optical frequencies ω₁ and ω₂ that are different from each other, and the difference (ω₁-ω₂) between the optical frequency ω₁ of the pump light and the optical frequency ω₂ of the Stoke light matches the angular frequency ω₀ of the natural oscillation of the molecules contained in the determination target.

As a result of the CARS process, when the oscillation mode of a specified molecular structure contained in the determination target is excited, the molecular oscillation interacts with probe light, which is a third laser light with an optical frequency ω₃, thereby generating the CARS light derived from the third-order nonlinear polarization.

The pump light can also be used as the probe light, and therefore the CARS light is generated under the condition ω₁ = ω₃. The CARS light generated by the detection target has an optical frequency that fulfills the condition ω_{CARS} = ω₁-ω₂+ω₃.

Accordingly, by detecting the CARS light emitted from the detection target, it is possible to non-invasively detect the presence of a specified molecular structure, e.g. a functional group, contained in the determination target. By repeatedly detecting the CARS light while changing the position where the determination target is irradiated with the excitation light, it is also possible to form an image of the distribution of the specified molecular structure in the determination target.

Since CARS light has a higher optical intensity than spontaneous Raman scattered light and the like, it is possible to perform the detection in a short time if a photoelectric converting element is used. Accordingly, not only does the time needed for the detection become shorter, but it is also possible to perform observation at a video rate. In this way, it is possible to detect not only the distribution of the specified molecular structure, but also changes in this distribution. Furthermore, by setting the band of the excitation light irradiating the determination target to be the infrared band, which causes little damage to cells, it is possible to observe cells of the determination target while these cells remain alive.

Furthermore, the CARS light occurring as a result of the nonlinear optical effect is generated in a very narrow region in which the excitation light is constricted by the lower objective lens 122. Therefore, the region that is a target for the CARS light detection is a region that is narrow in both the direction intersecting the optical axis of the excitation light and the direction parallel to the optical axis. Accordingly, observation of the determination target using CARS light has stereoscopically high resolution.

Therefore, the observation plane may be formed within the determination target using excitation light in or near the infrared band. Furthermore, by moving the observation plane sequentially in the depth direction of the determination target, it is possible to create an image reflecting the three-dimensional distribution of the specified molecular structure.

In the manner described above, known phenomena of a nonlinear optical effect occurring when the sample 101 is irradiated with coherent excitation light include a phenomenon by which, in addition to CARS light, Raman scattered light due to stimulated Raman scattering is generated, a phenomenon by which fluorescence is generated due to multi-photon excitation, a phenomenon by which harmonics such as second harmonics or third harmonics are generated, and the like. By detecting Raman scattered light among the lights generated by these nonlinear phenomena, it is possible to create an image that reflects the three-dimensional distribution of the specified molecular structure in the sample 101.

Even without using Raman scattered light, it is possible to create an image that reflects the three-dimensional structure of the sample 101. Accordingly, by suitably adjusting the wavelength of the laser light emitted by the laser device 130, the wavelength characteristics of the various filters, or the like, the determination device 100 can also be used when detecting radiation light generated from the sample 101 due to multi-photon excitation fluorescent light, second harmonics, third harmonics, stimulated Raman scattered light, or the like.

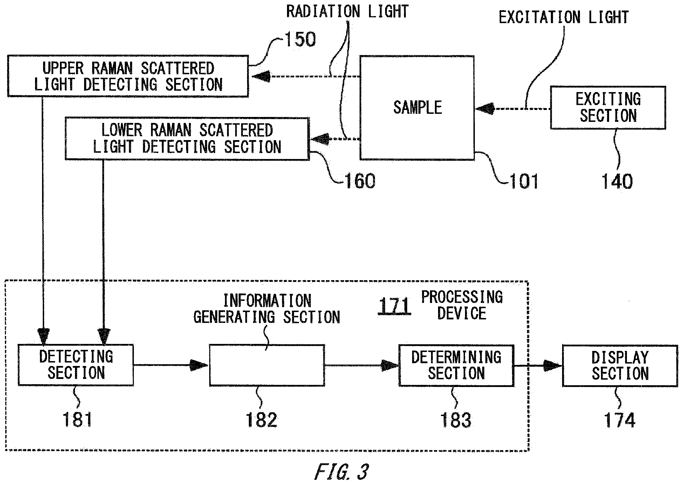

Fig. 3 is a block diagram of the determination device 100. As described with reference to Fig. 1, when the sample 101 is irradiated with the excitation light from the exciting section 140, Raman scattered light such as CARS light is generated in the sample 101. The Raman scattered light generated in the sample 101 is detected by the upper Raman scattered light detecting section 150 and the lower Raman scattered light detecting section 160.

The processing device 171, which is connected to the upper Raman scattered light detecting section 150 and the lower Raman scattered light detecting section 160 in the determination device 100, has a detecting section 181, an information generating section 182, and a determining section 183 implemented therein. The operations of the detecting section 181, the information generating section 182, and the determining section 183 are executed by a program stored in advance in the processing device 171 or a program loaded in the processing device 171 via a medium or a communication line.

The detecting section 181 acquires information reflecting the optical intensity such as a luminance value, for example, as the information concerning the detected Raman scattered light such as CARS light, from at least one of the upper Raman scattered light detecting section 150 and the lower Raman scattered light detecting section 160. Furthermore, the detecting section 181 stores the acquired optical intensity in association with a spatial position in the sample 101. The detecting section 181 may first discretize the acquired optical intensity, and then store this digitized value of the optical intensity in association with the position.

The information generating section 182 generates information obtained by, for example, quantifying the uniformity of the optical intensity values detected and acquired at a plurality of positions in the sample 101, based on the optical intensity values stored in association with the spatial positions. In the present embodiment, the uniformity refers to a state of the density of the detection targets within a two-dimensional or three-dimensional space in the sample 101 at a certain point in time. The uniformity becomes higher as the detection targets are dispersed in space and the density of the detection targets becomes uniform, and the uniformity becomes lower as the detection targets are condensed in space and variation of the density of the detection targets becomes greater. The determining section 183 can quickly determine the state relating to whether cells contained in the sample 101 that is the determination target are living or dead, using a simple process of comparing the quantified uniformity to a predetermined threshold value.

The processing device 171 outputs the determination results to the display section 174 to be displayed to the user, for example. Alternatively, the determination results may be accumulated in the processing device 171 in association with information identifying individual items in the sample 101 that are the detection targets.

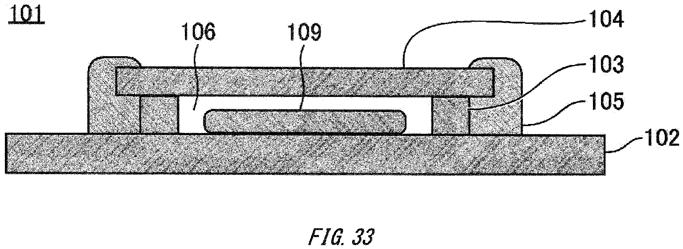

In order to decide the determination standards used by the determination device 100 by cross-referencing the state of the cells observed with the determination device 100 and a known state of cells, the sample 101 shown in Fig. 33 was created. Fig. 33 is a schematic cross-sectional view of the structure of the sample 101. The sample 101 includes a single-layer cell sheet 109 that is the determination target, a slide glass 102 that seals this cell sheet 109, a spacer 103, and a cover glass 104.

In the sample 101, the cell sheet 109 was sealed in a state where a sealing liquid 106 had been introduced between the slide glass 102 and the cover glass 104. Furthermore, the cell sheet 109 was sealed with the ring-shaped spacer 103 surrounding the cell sheet 109 being sandwiched between the slide glass 102 and the cover glass 104. A silicon rubber ring was used as the spacer 103.

Furthermore, in the sample 101, gaps between each of the slide glass 102, the spacer 103, and the cover glass 104 were sealed to be air tight by the sealing material 105. A commercial nail enamel was used as the sealing material 105.

Generally, in a state where a cell is dead, the cell membrane is damaged. The cell sheet 109 sealed in the sample 101 was living myoblasts, i.e. myoblasts that are maintained without the cell membranes being damaged, and was sealed in the single-layer cell sheet that is easily fluorescently stained. A gel sealing liquid including a staining reagent for detecting damage to the cell membrane was used as the sealing liquid 106. In this way, it was possible to restrict worsening of the observation environment in the sample 101 due to floating cells caused by the cells in the cell sheet 109 dying. Furthermore, due to the staining reagent mixed into the sealing liquid 106, there are cases of detecting fluorescent light and of detecting in color. The staining reagent intrudes into the cell nuclei whose cell membranes have been damaged due to the cell dying, thereby staining the proteins that are present in large amounts within the cell nuclei. Accordingly, by detecting that the inside of a cell nucleus has been stained by the staining reagent in the sample 101 from the outside with an optical microscope, it is possible to determine that the sealed cell sheet 109 is in a dead state.

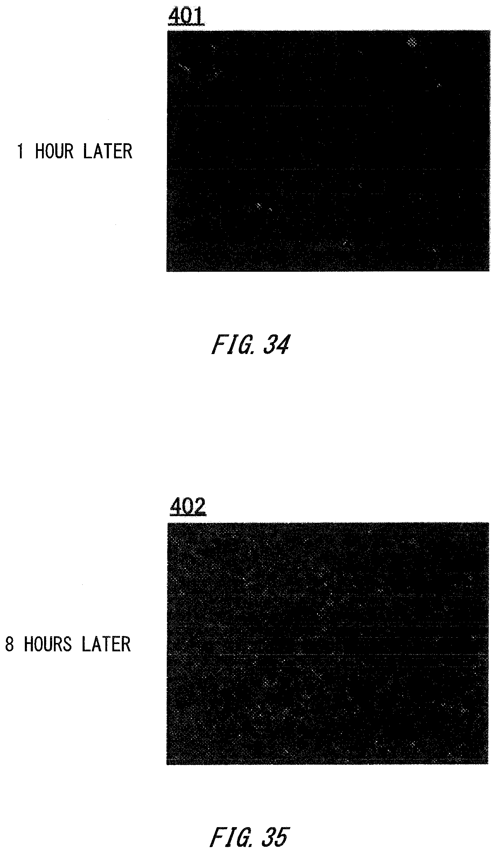

Fig. 34 is an optical micrograph 401 captured by observing the sample 101 manufactured in the manner described above after a time from 20 hours to 48 hours has passed since the sealing with the sealing material 105. The sample 101 was held constantly at 37°, and continued to be held at 37° using a heating device provided on the stage during the observation with the microscope. According to the optical micrograph 401, a small number of stained cells are present in the observation area, and it is judged that almost all of the cells contained in the cell sheet 109 are in the living state.

Fig. 35 is an optical micrograph 402 captured by observing the sample 101 described above after 8 hours have passed from the timing of the sealing with the sealing material 105, i.e. after 7 hours have passed from the state shown in Fig. 34. The sample 101 was held constantly at 37° from the previous observation, and continued to be held at 37° using a heating device provided on the stage during the observation with the microscope.

According to the optical micrograph 402, the number of stained cells has increased, and it is judged that there is a wide distribution of stained cells across the entire observation area. This indicates that, since the previous observation, many cells contained in the cell sheet 109 sealed in the sample 101 have changed state from being a cell that is living (sometimes referred to as a "live cell") to being a cell that is dead (sometimes referred to as a "dead cell"). The cause of death of these cells in the cell sheet 109 is thought to be a lack of oxygen in the sealing liquid 106 and the organic solvent contained in the sealing material 105.

In the present embodiment, a standard is used in which cells in a state where the cell membranes have been damaged such that the proteins within the cell nuclei are stained by the staining reagent in the manner described above are dead cells, and the determination device 100 determines the cells to be dead when this state is detected in the same sample 101 using the Raman scattered light generated by the CARS process. On the other hand, a standard is used in which cells in a state where the cell membranes remain undamaged such that the proteins in the cell nuclei are not stained by the staining reagent are live cells, and the determination device 100 determines the cells to be alive when this state is detected in the same sample 101 using the Raman scattered light generated by the CARS process.

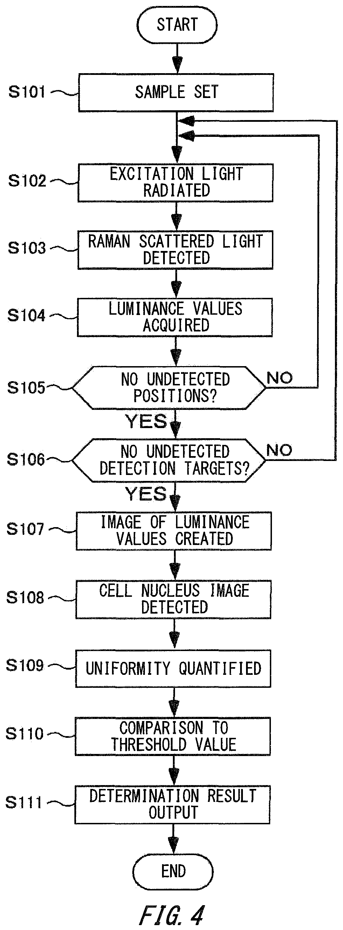

Fig. 4 is a flow chart of an exemplary determination procedure by the determination device 100. First, in the determination device 100, the user sets the sample 101 on the stage 110, for example (step S101). If the determination is to be made for a large number of samples 101, the setting of the samples 101 on the stage 110 and the transport of the samples 101 after the determination may be automated.

Next, the control section 170 of the determination device 100 irradiates the sample 101 set on the stage 110 with the excitation light from the exciting section 140. As a result, Raman scattered light is generated from the sample 101 due to the excitation light focused at the focal point of the lower objective lens 122 (step S102).

Next, the control section 170 detects Raman scattered light such as CARS light generated in the sample 101 that was irradiated with the excitation light, using at least one of the upper Raman scattered light detecting section 150 and the lower Raman scattered light detecting section 160 (step S103). Furthermore, the control section 170 acquires the Raman scattered light generated at the position irradiated with the excitation light using the detecting section 181, as the optical intensity value (step S104).

Next, the control section 170 checks whether there is a position at which detection has yet to be performed in the detection target region of the determination target (step S105). If there are remaining positions at which the detection has yet to be performed at step S105 (step S105: No), the control section 170 causes one of the stage scanner 111 and the galvanic scanner 141 to operate and move the sample 101 or the optical path of the excitation light such that the excitation light is focused at a position where detection has yet to be performed in the detection target region, and then returns the control to step S102.

In this way, the determination device 100 repeats the procedures of irradiating a position where detection has yet to be performed in the sample 101 with the excitation light (step S102) and acquiring another optical intensity value with the detecting section 181 (step S104). The control section 170 repeats the procedures from step S102 to step S105 described above until there are no more positions where detection has yet to be performed in the predetermined detection target area, and accumulates the plurality of detected optical intensity values in association with the positions in the sample 101 where these optical intensity values were respectively detected.

If there are no more positions where detection has yet to be performed in the detection target region of the sample 101 (step S105: YES), the control section 170 checks whether a detection target for which detection has yet to be performed is present in the sample 101 (step S106). Here, in a case where the proteins in the cells serving as the sample 101 are the initial detection target, for example, the detection target for which the detection has yet to be performed is lipids in these cells.

If the sample 101 is large, the control section 170 may further check whether there is another detection region remaining for which the detection has yet to be performed in one sample 101 and, if there is such a detection region, may repeat the detection until there are no more detection regions for which detection has yet to be performed. In this way, by selecting a location in the sample 101 that is judged to be prone to deterioration, e.g. a portion such as the edge or surface of the sample 101, and setting this location as the detection target region, it is possible to improve both the determination accuracy and the throughput.

At step S106, if it is judged that there is a detection target region remaining for which the detection has yet to be performed (step S106: NO), the control section 170 causes one of the stage scanner 111 and the galvanic scanner 141 to operate to move the sample 101 or the optical path of the excitation light in a manner to irradiate the detection target region for which detection has yet to be performed with the excitation light, and then returns the control to step S102. Accordingly, the control section 170 accumulates the plurality of optical intensity values detected for the next detection target region, in association with the positions in the sample 101 where the respective optical intensity values were detected.

At step S106, if it is judged that there are no remaining detection target regions for which detection has yet to be performed (step S106: YES), the control section 170 creates an image of the optical intensity values acquired for each detection target region in which optical intensity values are acquired (step S107). Here, creating an image in the determination device 100 refers to a set of information in which the plurality of optical intensity values for one detection target region are accumulated in association with the detection positions at which these optical intensity values were respectively detected, and does not necessarily refer to an image that can be viewed by the user. Furthermore, the detection positions included in this set of information may include three-dimensional positions.

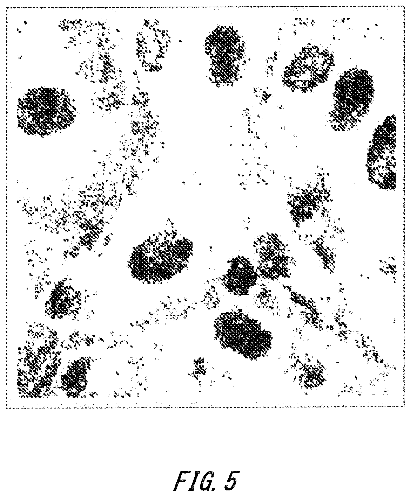

As an example of optical intensity values created as an image, luminance values converted into a two-dimensionally visible image are shown in Fig. 5. A cell sheet manufactured by culturing mammalian cells and formed of living cells, i.e. cells whose cell nuclei were not stained by the staining reagent, was used. Furthermore, with the wavelength of the excitation light generated by the exciting section 140 in the determination device 100, the band characteristics of the band-pass filters 154 and 164 of the upper Raman scattered light detecting section 150 and the lower Raman scattered light detecting section 160, and the band characteristics of the dichroic mirror 161 as shown in Table 1 below, CARS light was generated in the sample 101.

**Table 1:**

| | |
|---|---|
| LASER LIGHT SOURCE 131 | EMITTED LIGHT WAVELENGTH [nm]: 1120 - 1600 |
| LASER LIGHT SOURCE 132 | EMITTED LIGHT WAVELENGTH [nm]: 1064 |
| BAND-PASS FILTER 154 | TRANSPARENT BAND [nm]: 800 - 1010 |
| | BLOCKED BANDS [nm]: 350 - 770 |
| BAND PASS FILTER 164 | 1030-1650 |
| DICHROIC MIRROR 161 | TRANSPARENT BANDS [nm]: 380 - 750 |
| | 1064 - 1600 |
| | REFLECTED BAND [nm]: 800 - 1010 |

The image shown in Fig. 5 is obtained by creating an image of the luminance values of CARS light corresponding to the presence of proteins in the cell sheet described above. It should be noted that in the display method used here, portions with dark colors indicate regions with high luminance, i.e. positions where a large amount of proteins are present.

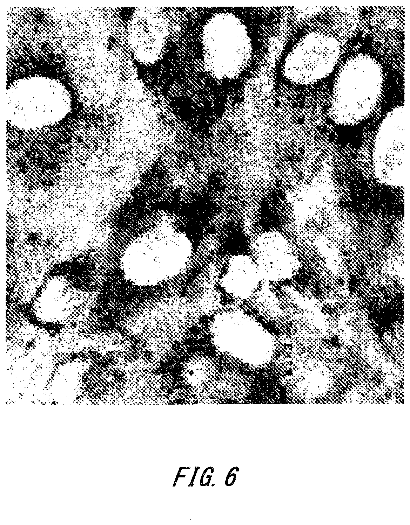

Fig. 6 shows an image obtained by creating an image of luminance values of CARS light corresponding to the presence of lipids in the same cell sheet. In this image as well, portions with dark colors indicate regions with high luminance, i.e. positions where a large amount of lipids are present. As shown in the drawings, in the cell sheet serving as the sample 101, it is judged that proteins and lipids are present in a substantially exclusive manner.

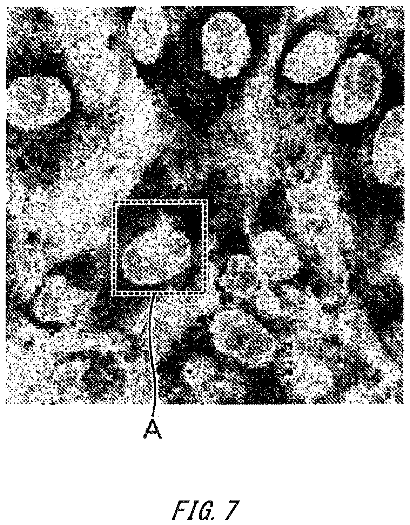

Fig. 7 is an image of a cell sheet created by combining the image shown in Fig. 5 and the image shown in Fig. 6, based on the difference between the luminance values of the radiation light generated from the proteins and the luminance values of the radiation light generated from the lipids. In this way, in the cell sheet serving as the sample 101, it is possible to clearly judge the regions occupied by the cell nuclei and easily make a distinction from simple depletion or the like.

In the present embodiment, proteins and lipids are used as the detection targets, but instead, as long as there are components that are contained in cells and have peaks in signal strength at a prescribed wavelength, in the same manner as proteins and lipids, it is possible to identify the component from the positions of these peaks, the signal strength, and the like. Therefore, if a component fulfills such conditions, this component other than proteins or lipids may be set as the detection target and the state of the cell may be determined based on the distribution of this component. A component that is localized within the cell nuclei is more preferable as the detection target.

If CARS light is being detected, a non-resonant background signal including refractive index information is unavoidably detected along with the CARS light itself. This non-resonant background signal acts as noise in the CARS light detection, thereby causing a decrease in the contrast and resolution of the generated observation image. Accordingly, when determining the state of the detection target using CARS light generated from the detection target, the effect of the non-resonant background signal is preferably eliminated.

When detecting CARS light generated in a cell, the C-H oscillation indicating the presence of lipids generates Raman scattered light with a high luminance that can be easily distinguished from the non-resonant background signal. Accordingly, in the information image generated by detecting CARS light, for example, generated by lipids, it is possible to detect the range in which lipids are present regardless of the presence of the non-resonant background signal.

Furthermore, since proteins and lipids are present in an exclusive manner in the cells, by obtaining the difference between the image generated with CARS light generated due to the presence of proteins and the image generated with CARS light generated due to the presence of lipids, it is possible to restrict the effect of the non-resonant background signal and include the presence of proteins in the information image. In this way, by obtaining the difference between the information about proteins and the information about lipids in the cell nuclei in which the lipids are not present or exist in an extremely small amount compared to the proteins, it is possible to acquire information concerning the proteins in the cell nuclei from which the non-resonant background signal has been eliminated.

In this way, if the detection target set as the objective is proteins, it is possible to improve the detection accuracy for the proteins by also using lipids as a detection target. It should be noted that if the detection target set as the objective is lipids, it is sufficient to set lipids directly as the detection target. Furthermore, by setting yet another component as a detection target, it is possible to further improve the detection accuracy for lipids.

When combining these images, the proteins and lipids may be combined while having different colors applied thereto. This coloring does not involve dying the cell sheet itself, and therefore the ease of identification with staining can be realized without anything invading the living cell sheet. With reference to Fig. 4 again, as a result of the image processing described above, it is possible to detect the region in the image occupied by the cell nuclei that are the true targets of the determination in the sample 101 (step S108).

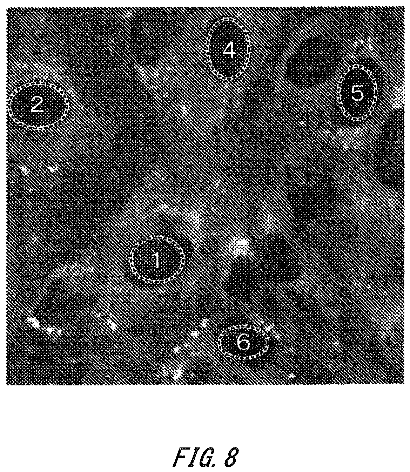

Fig. 8 is an image showing the region occupied by the cell nuclei extracted from the cell sheet for which an image was created, as described above. As shown in the drawing, upon judging the positions and widths of the cell nuclei, the control section 170 quantifies the uniformity of the luminance values in the image obtained at step S107 for a region including any one of the cell nuclei (step S109).

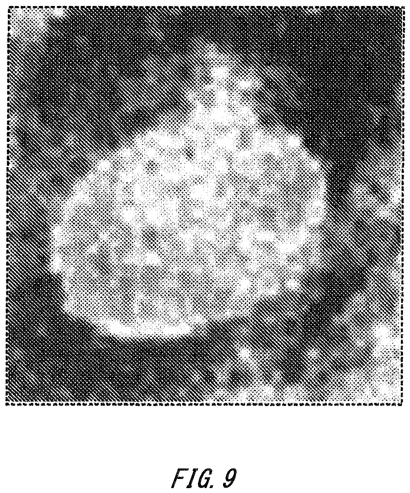

Fig. 9 is an enlarged view of a region including one cell nucleus indicated by the dotted line A in Fig. 7. As shown in the drawing, in the cell nucleus of a living cell, the luminance values indicating the presence of proteins are uniform, and the uniformity of the optical intensity values is judged to be high.

In the image shown in Fig. 9, the cell nucleus occupies a large portion of the image. Accordingly, by performing the quantification process on the region shown in Fig. 9, it is possible to quantify the uniformity of the detection target in the cell nucleus within the region of this image (step S109). In the present embodiment, the uniformity of the presence of proteins in the cell nucleus is quantified.

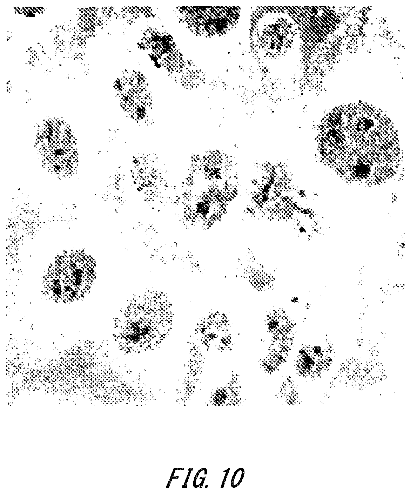

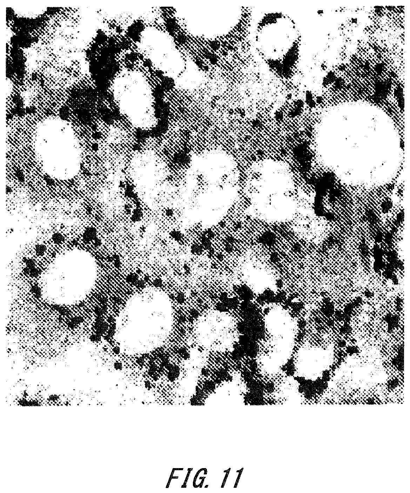

Fig. 10 shows an image created by acquiring luminance values, as one example of optical intensity values of CARS light corresponding to the presence of proteins, in a cell sheet formed from dead cells. Displaying dark portions as regions with high luminance values, i.e. positions where there is more proteins present, is the same as in the other drawing from Fig. 5. Fig. 11 shows an image based on the luminance values of CARS light corresponding to the presence of lipids, in the same cell sheet. In this drawing as well, dark portions indicate regions with high luminance values, i.e. positions where there are more lipids present.

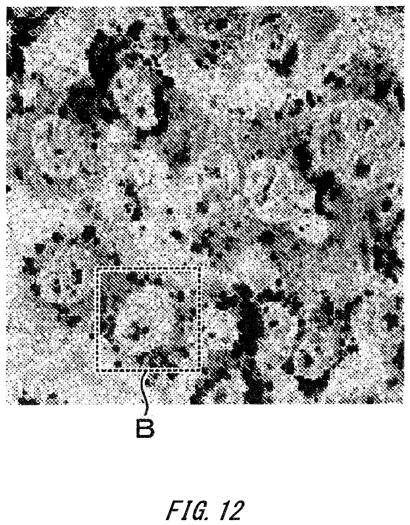

Fig. 12 is an image of a cell sheet generated by combining the image shown in Fig. 10 and the image shown in Fig. 11. When combining these images as well, the proteins and the lipids may have different colors applied thereto. In this way, in the cell sheet serving as the sample 101, it is possible to clearly judge the regions occupied by the cell nuclei and easily make a distinction from simple depletion or the like. In this way, the region of the image occupied by cell nuclei that are the determination targets in the sample 101 are detected (step S108).

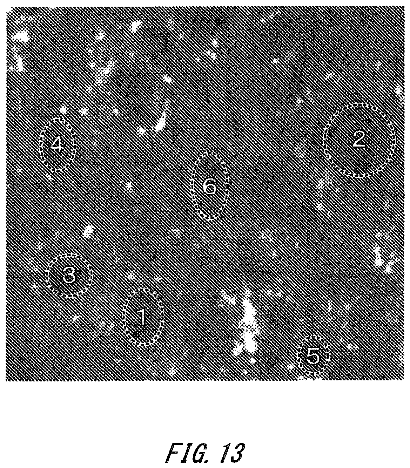

Fig. 13 is an image showing the regions occupied by the cell nuclei extracted from the cell sheet for which the image was created. As shown in the drawing, upon judging the positions and widths of the cell nuclei, based on the luminance values detected from a region including any one cell nucleus, the control section 170 determines the living/dead state of the cells including these cell nuclei.

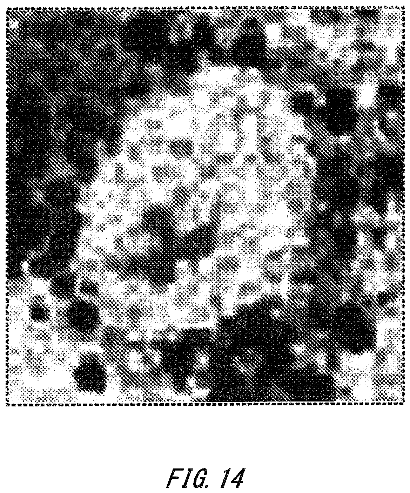

Fig. 14 is an enlarged view of a region including one cell nucleus indicated by the dotted line B in Fig. 12. As shown in the drawing, in the cell nucleus of a dead cell, there is variance among the luminance values indicating the presence of proteins, there are localized regions where the proteins are present in large amounts or in small amounts, and the uniformity of the optical intensity values is judged to be low.

In other words, in the cell nucleus of a living cell, the proteins are dispersed within the cell nucleus with an approximately uniform density, and therefore the differences between luminance values at a plurality of positions are small and the uniformity of the proteins is high. On the other hand, in the cell nucleus of a dead cell, the proteins are condensed in the cell nucleus and there is variation in the density of the proteins, and therefore the differences between luminance values at a plurality of positions are large and the uniformity of the proteins is low.

In the image shown in Fig. 14, the cell nucleus occupies a large portion of the image. Accordingly, by performing the quantification process on the region shown in Fig. 14, it is possible to quantify the uniformity of the detection target in this image (step S109). In the present embodiment, the uniformity of the presence of proteins in the cell nucleus is quantified.

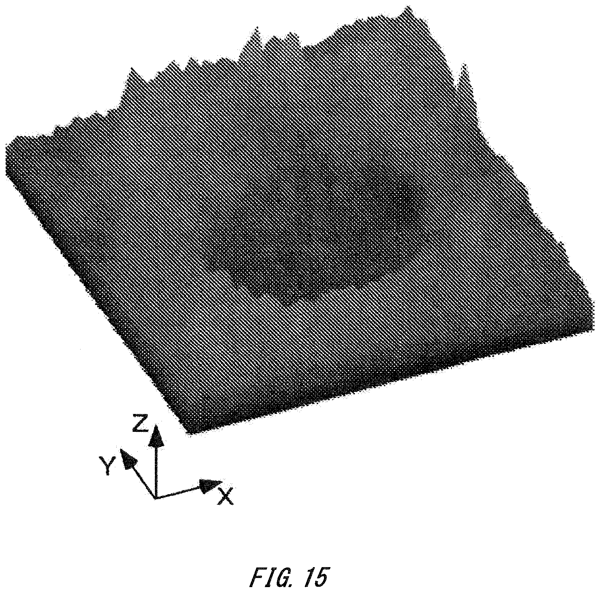

Fig. 15 is a drawing for describing one method for quantifying the uniformity of the presence of proteins in the determination target, performed at step S109 of the procedure shown in Fig. 4. Fig. 15 is a drawing obtained by creating a stereoscopic image of the luminance values acquired for live cells and includes the cell nucleus shown in Fig. 9.

The plane indicated by the X and Y arrows in the drawing corresponds to the plane of Fig. 9. Furthermore, the direction indicated by the Z arrow in the drawing indicates the luminance value at each position in the X-Y plane. It should be noted that, as described in Figs. 5 and the like, the luminance values in the image created at step S107 are more negative when these luminance values are higher. Accordingly, the -Z side of the Z axis in Fig. 15 represents the height of the luminance values corresponding to the presence of proteins.

The information generating section 182 quantifies the uniformity of the presence of proteins by calculating the ratio of the maximum peak value of the luminance values to the average value of the luminance values, among the luminance values in the created image. As an example, in a living cell, the uniformity of the presence of proteins is high. Therefore, when a threshold value, e.g. 5 times, is set for the ratio of the peak value of the luminance values to the average and the same ratio is calculated for an unknown determination target, if this ratio is less than 5, the determining section 183 can determine that this determination target is living (step S110).

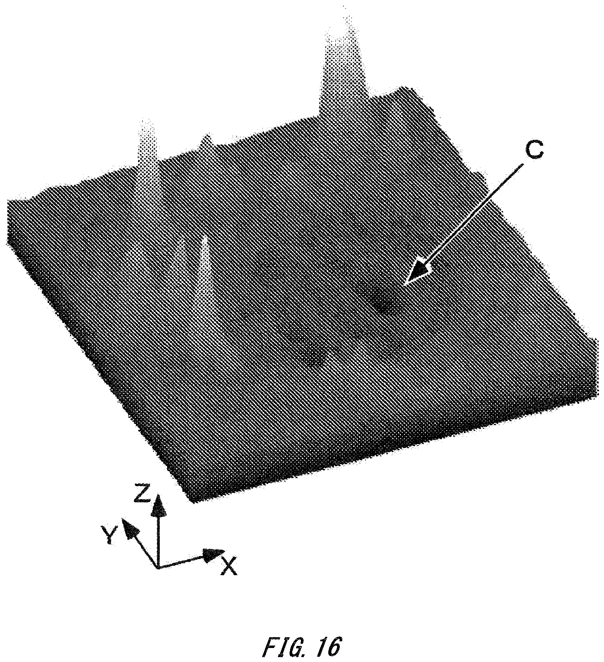

Fig. 16 is a drawing obtained by creating a stereoscopic image of the luminance values acquired for a dead cell having the cell nucleus shown in Fig. 14. In the drawing, X, Y, and Z have the same meaning as in Fig. 15.

The information generating section 182 quantifies the uniformity as a ratio of the maximum peak value indicated by the arrow C in the drawing to an average value of the luminance values, among the luminance values in the created image, and compares this ratio to a predetermined threshold value (step S110). In a dead cell, the uniformity of the presence of proteins is low, and therefore large peaks occur in the image of the drawing, and the peak value of the luminance values is greater than 5 times the average value, which is an example of the threshold value. Accordingly, the determining section 183 can determine that this determination target is dead.

Furthermore, the information generating section 182 may calculate the average value of the ratio of the maximum peak value to the minimum peak value in one region of a biological cell that is the determination target and the ratio of the maximum peak value to the minimum peak value in a neighboring region of this one region, and set this average value as a contrast evaluation value for evaluating the contrast in the image (step S109). The contrast evaluation value generated in this way is compared to a predetermined threshold value for the contrast evaluation value (step S110), thereby making it possible to eliminate the effect of noise and easily and reliably make the determination for an unknown determination target.

The determination result obtained at step S110 is output to the user, for example (step S111). The output of the determination result may be displayed on the display section 174 to inform the user and various markings may be made in the determination result, for example. Furthermore, if the loading and unloading of the determination target in to or out of the determination device 100 is automated, the determination target may be moved to a different output destination according to the determination result. In this way, even when there are a large number of determination targets, it is possible to accurately determine the living/dead state and safely use the cell sheet, for example.

In the manner described above, when quantifying the uniformity using the ratio of the peak value to the average value, the information generating section 182 may perform a process to reduce the noise of the luminance values. The noise reduction can be performed by replacing each luminance value associated with a respective position in the spaces shown in Figs. 15 and 16 with a weighted average of the luminance values within a predetermined region including this position, for example. In this way, it is possible to make outlying values among the luminance values uniform and improve the determination accuracy of the determining section 183.

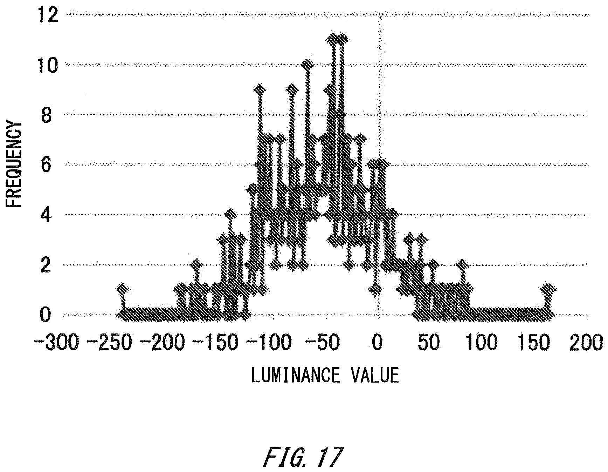

Fig. 17 is graph showing a histogram of the luminance values acquired from living cells including the cell nucleus shown in Fig. 9 and the occurrence frequency thereof, and shows another example of step S109 for quantifying the uniformity. As described in Fig. 5 and the like, the luminance values in the image created at step S107 are more negative when these luminance values are higher. Accordingly, the left side of the origin on the horizontal axis in Fig. 17 represents the height of the luminance values corresponding to the presence of proteins as negative values.

As described above, in the living cells shown in Figs. 5 to 9, the uniformity of the presence of proteins in the cell nuclei is high. Therefore, in the histogram shown in the drawing, the ratio of the maximum value on the horizontal axis at which the absolute value of the luminance is at a maximum to the average value of the luminance values is relatively small.

In the example shown in the drawing, the uniformity of the presence of proteins is quantified as the ratio (4.7) of the value (-243.3) corresponding to the maximum luminance to the average value (-51.1) (step S109). Accordingly, the determining section 183 can easily make the determination for an unknown determination target, by comparing the quantified uniformity to the predetermined threshold value of 5, for example (step S110).

As shown in Fig. 8, for each of the plurality of cell nuclei 1 to 6 (cell nucleus 3 is not shown in the drawing) that are living cells, the uniformity of the luminance was quantified using the method described above. The uniformity of the presence of proteins in each cell nucleus quantified in this manner is shown in Table 2 below. As shown below, for every cell nucleus, the ratio of the maximum value to the average value among the luminance values is less than 5, which is an example of the threshold value.

**Table 2: Cell Nuclei of Fig. 8**

| | |
|---|---|
| Cell Nucleus 1: | 4.7 |
| Cell Nucleus 2: | 3.7 |
| Cell Nucleus 3: | 4.1 |
| Cell Nucleus 4: | 3.9 |
| Cell Nucleus 5: | 2.5 |
| Cell Nucleus 6: | 2.8 |

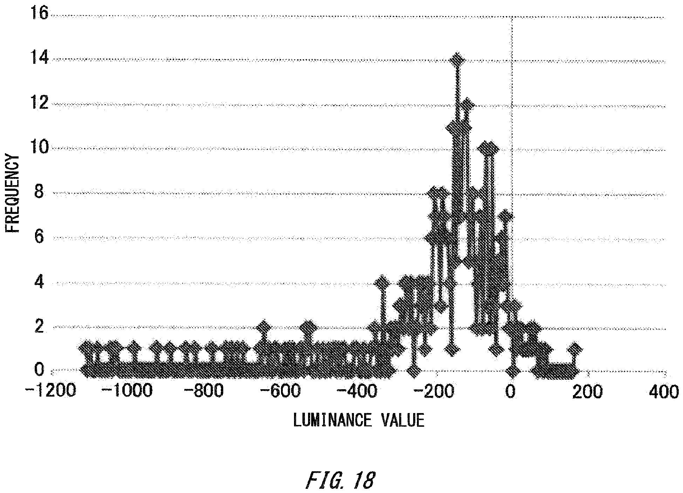

Fig. 18 is a graph showing a histogram of the luminance values acquired from dead cells including the cell nucleus shown in Fig. 13 and the occurrence frequency thereof. In Fig. 18 as well, the luminance values in the image created at step S107 are more negative when these luminance values are higher. Accordingly, the left side of the origin on the horizontal axis in Fig. 18 represents the height of the luminance values corresponding to the presence of proteins as negative values.

As described above, in the dead cells shown in Figs. 10 to 14, the uniformity of the presence of proteins in the cell nuclei is low. Therefore, in the histogram shown in the drawing, the ratio of the maximum value on the horizontal axis at which the absolute value of the luminance is at a maximum to the average value of the luminance values is relatively large.

In the example shown in the drawing, the ratio (5.6) of the value (-1113.3) corresponding to the maximum luminance to the average value (-198.4) is greater than 5, which is an example of the threshold value described above. Accordingly, the determining section 183 can determine that the unknown determination target is a cell in the dead state, by comparing the quantified uniformity to this threshold value (step S110).

Table 3 below shows values obtained by quantifying the uniformity of the luminance using the method described above, for each of the plurality of cell nuclei that are dead cells shown in Fig. 13. As shown below, for every cell nucleus, the ratio of the maximum value among the luminance values and the average value is greater than the threshold value described above.

**Table 3: Cell Nuclei of Fig. 13**

| | |
|---|---|
| Cell Nucleus 1: | 17.5 |
| Cell Nucleus 2: | 6.1 |
| Cell Nucleus 3: | 5.6 |
| Cell Nucleus 4: | 7.8 |
| Cell Nucleus 5: | 6.5 |
| Cell Nucleus 6: | 6.8 |

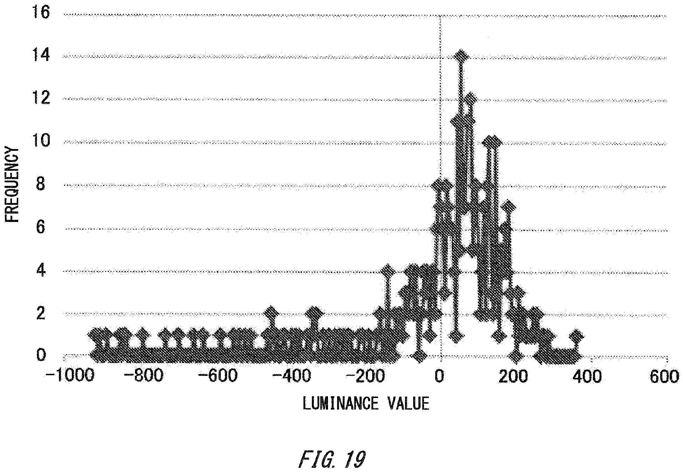

Fig. 19 is a graph obtained by shifting the average value of the luminance values in the graph shown in Fig. 18 to the position of the origin on the horizontal axis, and shows another example of step S109 for quantifying the uniformity. Shifting the luminance value histogram in this way can only mean calculating each of a first value obtained by subtracting the average value from the maximum value among the luminance values and a second value obtained by subtracting the average value from the minimum value among the luminance values. Accordingly, it is possible to quantify the uniformity of the presence of proteins in the sample 101 using the ratio of the maximum value to the minimum value in the graph shown in Fig. 19.

In the example of the drawing, a ratio of 2.5 can be calculated as the ratio of the maximum value on the positive side to the maximum value among the luminance values on the negative side using a simple process, thereby quantifying the uniformity of the presence of proteins in the dead cells. When the same process is performed for the luminance values acquired from living cells shown in Fig. 17, the information generating section 182 can calculate a ratio of 0.8 as the ratio of the maximum value on the positive side to the maximum value among the luminance values on the negative side, thereby quantifying the uniformity of the presence of proteins in the living cells. Accordingly, by comparing this ratio to a predetermined threshold value, the determining section 183 determines the living/dead state of the unknown sample 101 (step S110).

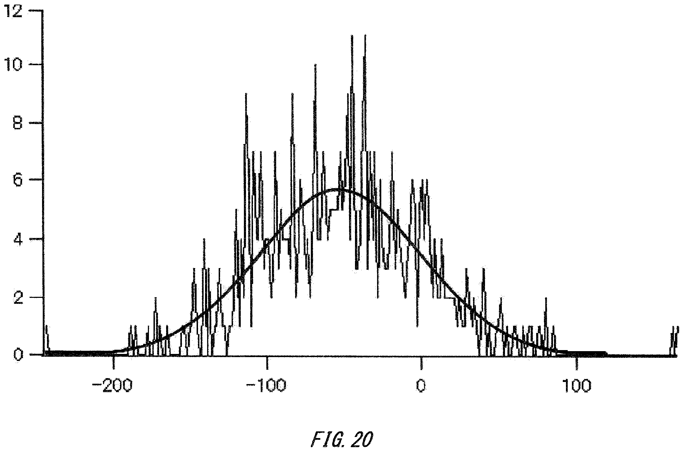

Fig. 20 is a graph for describing another method for quantifying the uniformity of the presence of proteins in the determination target at step S109. As described in Fig. 5 and the like, the luminance values in the image created at step S107 are more negative when these luminance values are higher. Accordingly, the left side of the origin on the horizontal axis in Fig. 20 represents the height of the luminance values corresponding to the presence of proteins.

The thin line with the complex bending in the graph of the drawing indicates the histogram of the luminance values acquired for a living cell having the cell nucleus shown in Fig. 9. Furthermore, in Fig. 20, the thick curved line with the gradual slope superimposed on the histogram described above indicates a Gaussian function obtained as a result of performing Gaussian fitting on the histogram of the luminance values described above. In this way, by applying Gaussian fitting to the luminance values acquired by the detecting section 181 (at step S104), the information generating section 182 can quantify the uniformity of the presence of proteins in the sample 101 (step S109).

Furthermore, the information generating section 182 can quantify the luminance values that have undergone the quantification process, as an integrated value of the frequencies up to the luminance value at which the Gaussian function has a predetermined slope, for example. As described above, the uniformity of the presence of proteins in a living cell is high. Therefore, the peak of the Gaussian function obtained from the quantification process is positioned at substantially the center of the distribution and traces a curve close to the normal distribution. Accordingly, in the example of the drawing, the integrated value of the frequency up to the luminance value where there is the predetermined slope described above, e.g. dy/dx < 0.1, is small.

Therefore, when the same process is performed for an unknown determination target, if the comparison is made in advance to a threshold value of 10, for example, and the obtained integrated value for the frequency is less than 10, the determining section 183 can determine that this determination target is alive (step S110). In this way, when the excitation light is radiated, it is possible to detect the optical intensity of the Raman scattered light generated by the detection target contained in the biological cell, generate information reflecting the spatial uniformity of the detection target based on the detection results, and determine the state of the cell based on the generated information.

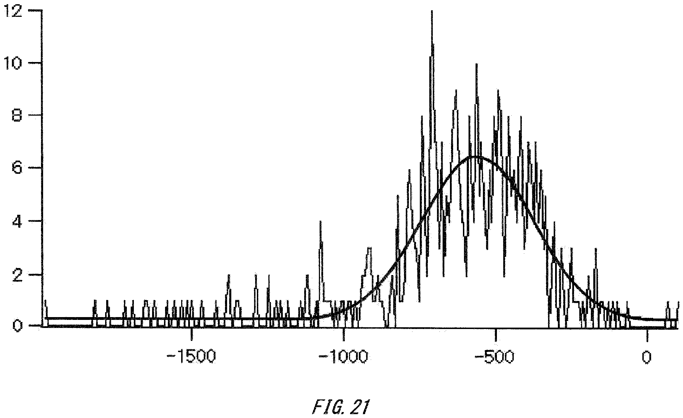

Fig. 21 shows a histogram of luminance values acquired for a dead cell having the cell nucleus shown in Fig. 14. In Fig. 21 as well, the thick curved line with the gradual slope superimposed on the histogram described above indicates a Gaussian function obtained as a result of performing Gaussian fitting on the histogram described above.

As described above, the uniformity of the presence of proteins in the cell nucleus of a dead cell is low. Therefore, the peak of the Gaussian function obtained from the quantification process traces a curve that is distanced from the center of the distribution range. Accordingly, in the example of the drawing, the integrated value of the frequency up to the luminance value at which the slope is the predetermined slope described above is greater than or equal to 10. Accordingly, when the same process is performed for an unknown determination target, if the obtained integrated value for the frequency is greater than or equal to the threshold value of 10 described above, the determining section 183 can determine that this determination target is dead.

The quantification method of the Gaussian function obtained by applying Gaussian fitting to the histogram of the acquired luminance values is not limited to calculating the integrated value of the frequency such as described above. For example, the information generating section 182 may quantify the uniformity as the ratio of the luminance value at which the peak of the Gaussian function is formed to the average value of the luminance values.

In a living cell, the uniformity of the presence of proteins in the cell nucleus is high, and therefore the ratio described above acquired from the quantification is small, e.g. less than 5. In contrast, for the luminance values acquired by the detection from a dead cell, the ratio described above is greater than or equal to 5. Accordingly, by quantifying the uniformity of the presence of proteins as the ratio of the luminance value at which the Gaussian function forms the peak to the average value, the determining section 183 can determine the living/dead state of the determination target by comparing this ratio to the threshold value.

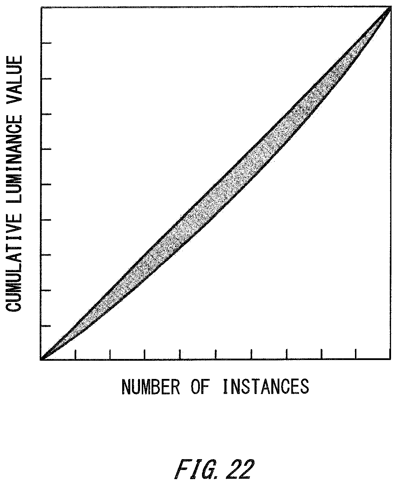

Fig. 22 is a graph of a Lorentz curve obtained by performing the quantification process of integrating the luminance in a region including one cell nucleus in a cell sheet containing the living cell shown in Fig. 9, and shows another example of step S109 for quantifying the uniformity. The horizontal axis in this graph indicates the number of occurrences of pixels for which the acquired luminance values are equal. The vertical axis in this graph indicates a cumulative value of the acquired luminance values.

In the graph in the drawing, the vertical axis is normalized such that the maximum is 1. Furthermore, the straight dotted line in the drawing is a line of perfect equality with a slope of 45 degrees serving as a reference index of the Lorentz curve calculated from the cumulative luminance value. In the graph of the drawing, the deviation of the Lorentz curve from the line of perfect equality can be quantified by the area of the region indicated by the shading in the drawing (step S109). Accordingly, by comparing the value of this area to a predetermined threshold value, it is possible to determine whether the cell in the sample 101 is in the living state (step S110).

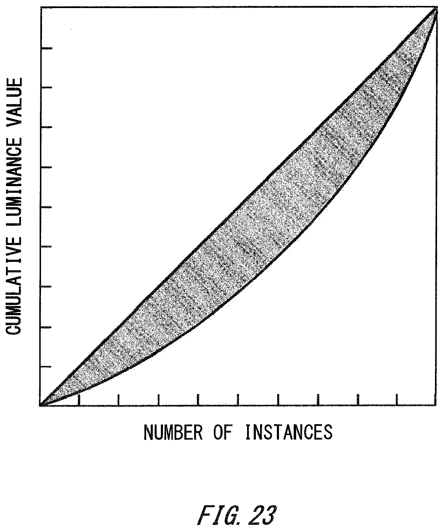

Fig. 23 is a graph showing the Lorentz curve obtained by quantifying the distribution of luminance values of a region including one cell nucleus in step S109, for a cell sheet including dead cells shown in Fig. 14. As shown in the drawing, the deviation of the Lorentz curve from the line of perfect equality shown by the shading in the drawing can be quantified by the area of the region indicated by the shading in the drawing (step S109). Furthermore, the area of the shaded region shown in Fig. 23 is clearly greater than that of the graph shown in Fig. 22, and by comparing the area of this region to a predetermined threshold value, it is possible to determine the information concerning whether the cell contained in the sample 101 is living or dead (step S110).

In this way, the steps from step S101 to S109 are performed for the unknown determination target to quantify the uniformity of the presence of proteins in this determination target, after which this numerical value is compared to a predetermined threshold value (step S110), thereby enabling a quick and accurate determination concerning whether the cell that is the determination target is living, dead, or not yet dead but dying.

The threshold value used in step S110 described above can be determined by performing steps S101 to S109 on a sample 101 for which the living/dead state has been judged in advance. For example, by performing steps S101 to S109 on a cell sheet that has been judged in advance to be living, it is possible to know a range of numerical values occurring when the uniformity of the presence of proteins in the cell nucleus of a living cell is quantified. Accordingly, by determining a numerical value that defines this range of numerical values to be the threshold value, it is possible to determine a threshold value for judging that a cell is alive.

Similarly, a threshold value may be obtained that occurs when judging that a cell is dead by performing steps S102 to S109 on a cell sheet in which the cells were judged in advance to be dead. The determination result for the living/dead state of a cell is not necessarily limited to one of living and dead. Time is needed for a biological cell to transition from the living state to the dead state, i.e. there can be an intermediate state from when a cell begins to die to when the cell is completely dead. Accordingly, in addition to the threshold value for determining that a cell is alive, a threshold value for determining that a cell is dead may be provided, and a state between these threshold values may further be judged as a state relating to being alive or dead.

Instead of the determination method described above, the living/dead state of a cell may be determined using another method. For example, a cell can be judged to be in the dead state if the length of a flat portion of the histogram described above is greater than a prescribed value, and a cell can be judged to be in the living state if the length of a flat portion of the histogram described above is less than a prescribed value. The threshold value for the length of the flat portion for judging whether a cell is living or dead may be determined by observing a sample that has been judged in advance to be living or dead. Furthermore, the threshold value may be adjusted in accordance with the intended use of the cell.

As another method for determining the living/dead state of a cell, a half-width of a crest including the peak value of the histogram described above may be compared to the length of the flat portion of this histogram. In this case, the cell can be determined to be in the dead state if the length of the flat portion is greater, and the cell can be determined to be in the living state if the length of the flat portion is smaller.

Furthermore, a cell can be determined to be in the dead state if the position of the luminance value where the frequency peaks in the histogram described above is outside of a predetermined range including the center of the total measurement range, and the cell can be determined to be living if the position of the luminance value where the frequency peaks is within this range.

Furthermore, a cell that is the determination target can be determined to be in the living state if the position of the peak of the histogram is within a predetermined first range including the center of the total measurement range, and this cell can be determined to be in the dead state if the position of this peak is within a predetermined second range in a region different from the first range described above. Furthermore, the cell that is the determination target can be determined to be in the intermediate state of dying between being living and dead if the position of this peak is in a range outside of both the first range and the second range described above.

Yet further, the shapes of histograms detected by observing cells whose states have been judged in advance can be stored in advance for each state of a cell including the living state, the dead state, and the intermediate state between being living and dead, and the shape of a histogram detected from a cell that is the determination target can be compared to the shapes of the stored histograms to determine the living/dead state of the cell from the matching rate of the shapes.

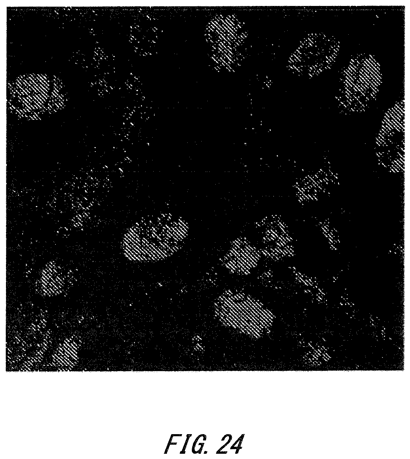

Figs. 24 to 28 are drawings for describing yet more determination methods. Fig. 24 shows an observation image obtained by observing cells that have been judged in advance to be living, with the determination device 100. In the observation image of the drawing, the distribution of the proteins in the nuclei of the cells that are the determination targets can be seen using the CARS process. As shown in the drawings, the proteins are distributed uniformly in the regions corresponding to the cell nuclei in the observation image of the living cells.

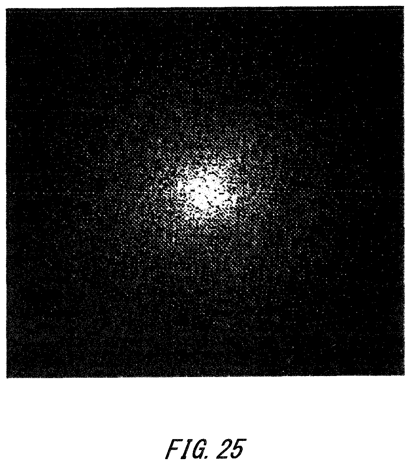

Fig. 25 is an image showing the results of performing a two-dimensional Fourier transform on the observation image shown in Fig. 24. When performing the two-dimensional Fourier transform on the observation image, the two-dimensional Fourier transform is performed after the observation image has been made monochromatic using a primary color separation or grayscale conversion. The image obtained as a result of the two-dimensional Fourier transform in this way is referred to below as the converted image. As shown in the drawing, in the converted image acquired from the observation image in which the proteins are distributed uniformly, a region with high luminance is formed gathered near the center. The region with high luminance in the converted image is referred to below as the Fourier region.

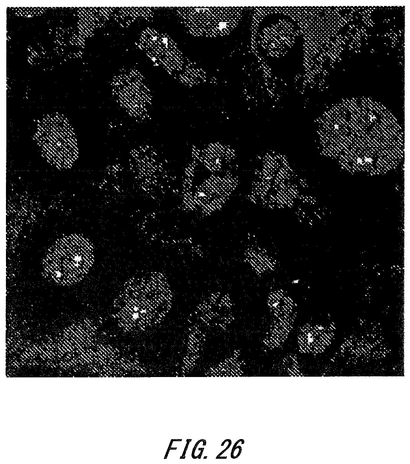

Fig. 26 shows an observation image obtained by observing a cell that was judged in advance to be dead, with the determination device 100. In the observation image of the drawing, regions with extremely high luminance can be seen scattered within the nuclei of the cells that are the determination targets. As shown in the drawing, regions in which the proteins are distributed in a localized manner appear in the observation image in the regions corresponding to the cell nuclei in the observation image of the dead cells.

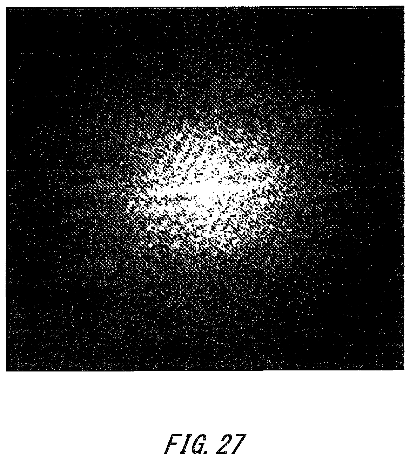

Fig. 27 shows a converted image obtained by performing a two-dimensional Fourier transform on the observation image shown in Fig. 26. With the converted image of this drawing as well, before the conversion process, the observation image was made monochromatic using a primary color separation or grayscale conversion and then the two-dimensional Fourier transform was performed. As shown in the drawing, in the converted image acquired from the observation image in which the proteins are distributed in a localized manner, the region with high luminance is formed with a greater spread than in the image shown in Fig. 25.

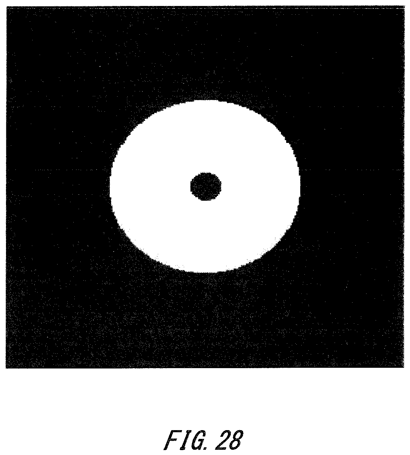

Fig. 28 is an image indicating the threshold value when determining the living/dead state of a cell that is an observation target, based on a converted image obtained in the manner described above. In the image of the drawing, a boundary with a region having low luminance is formed at each of the inside and the outside of the region with high luminance. Therefore, in the drawing, the region with high luminance forms a ring shape with a constant width. Below, the image of this drawing is referred to as the threshold value image, and the ring-shaped region with high luminance in the threshold value image is referred to as the threshold value region.

Here, in a comparison between the threshold value image and the converted images shown in Figs. 25 and 27, the overlap between the Fourier region of the converted image shown in Fig. 25 and the threshold value region in the threshold value image is 0.37 (relative placement). Furthermore, the overlap between the Fourier region of the converted image shown in Fig. 27 and the threshold value region in the threshold value image is 0.52 (relative placement).

As described above, if a threshold value of 0.45 is set for the value of the overlap between the Fourier region of the converted image and the threshold value region of the threshold value image as a result of observing cells that have been judged to be living and cells that have been judged to be dead a plurality of times, the cell that is the observation target can be determined to be living if this value is less than or equal to 0.45 and the cell can be determined to be dead if this value is greater than 0.45. In this way, by using the determination device 100 to quantify non-uniformity of the proteins in the cell nuclei via the quantification process after making the protein distribution in the nuclei visible through the CARS process, and to then compare the numerical value to the predetermined threshold value, it is possible to determine whether the cells are living or dead and to evaluate the quality of the cell sheet.

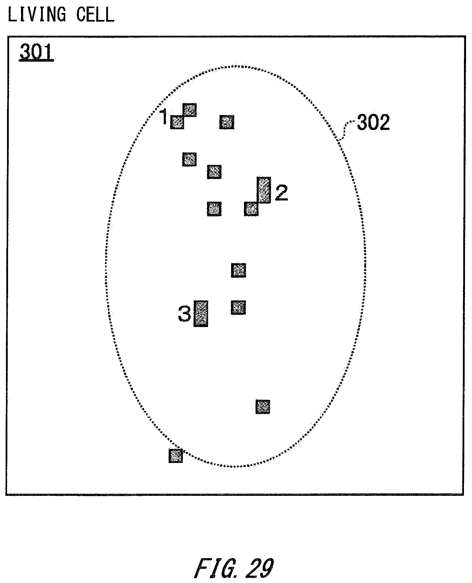

Fig. 29 shows another example of an information image indicating information relating to the uniformity of proteins in the cell nucleus of a biological cell. The information image 301 in the drawing was obtained in the following manner.

First, a biological cell that was judged in advance to be a living cell was mounted in the determination device 100 as the sample 101 and CARS light generated from the sample 101 as a result of being irradiated with excitation light was measured with the detecting section 181. The excitation light was selected to have a wavelength causing CARS light to be generated by the molecules of the proteins included in the sample 101. In this way, the observation image reflecting the distribution of proteins in the sample 101 was obtained.

Next, the observation image obtained by the detecting section 181 was processed by the information generating section 182. In the information generating section 182, the observation image was binarized using 2σ in the luminance distribution of the observation image as a threshold value, thereby generating a binary image showing the regions in which the CARS light luminance is high.

Furthermore, in the determination device 100, the sample 101 was irradiated with radiation light having a different wavelength than the wavelength that generated the CARS light described above. At this state, the wavelength of the radiation light irradiating the sample 101 was selected to be a wavelength that does not cause a nonlinear optical effect with the proteins, lipids, and the like contained in the sample 101. Accordingly, Raman scattered light was not emitted from the sample 101.

Instead, by detecting the radiation light transparently passed through the sample 101 with the detecting section 181, an observation image reflecting the refractive index distribution in the sample 101 was acquired with the detecting section 181. In this way, the contour of the cell nucleus of the biological cell is detected in the observation image and, when detecting CARS light, it is possible to select the CARS light generated within the cell nucleus. Accordingly, it is possible to reduce the effect of the non-resonant background signal caused by water molecules or the like present around the biological cell serving as the determination target, and to detect in a focused manner the proteins contained in the cell nucleus of the biological cell that is the evaluation target.

Next, the binary image described above derived from CARS light and the contour image obtained with the radiation light that does not cause a nonlinear optical effect were stacked, to obtain the information image 301 shown in Fig. 29. In the information image 301, the contour of the cell nucleus region 302 and the binary image with the bright spots that are regions with high CARS light luminance are shown as being stacked.

Next, in order to improve the S/N ratio in the information image, the information generating section 182 calculates the area of high-luminance pixel groups in which two or more pixels are continuous, while omitting the pixels that exist independently. In the example of the drawing, a plurality of high-luminance pixel groups numbered 1 to 3 in the drawing are distributed within the contour of the cell nucleus region 302.

The information generating section 182 calculates the total value of the areas of the high-luminance pixel groups 1 to 3 and calculates the percentage of the entire area of the cell nucleus region 302 occupied by this total value. In the example of the drawing, in the cell nucleus with a size corresponding to 488 pixels, the high-luminance pixel groups indicating regions with high concentrations of proteins occupied 7 pixels, which means that the high-luminance pixel groups occupy 1.4% of the total area of the cell nucleus region 302. The calculated value is transferred to the determining section 183 as the information obtained by quantifying the uniformity of the proteins in the sample 101.

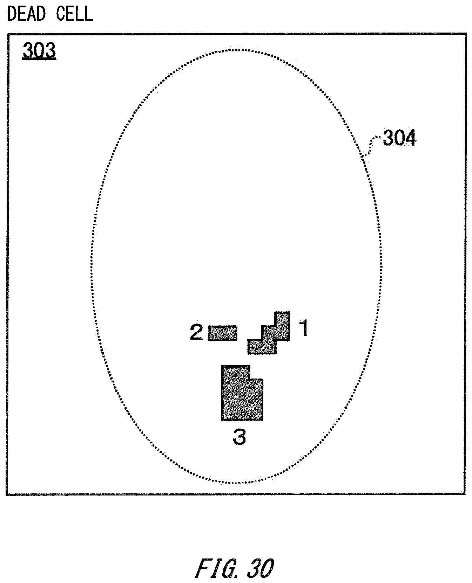

Fig. 30 shows yet another example of an information image showing information relating to the uniformity of the proteins in a biological cell. The information image 303 in the drawing was obtained by mounting a biological cell that was judged in advance to be a dead cell in the determination device 100 as the sample 101 and performing the same process as the method used to obtain the information image 301 shown in Fig. 29. As shown in the drawing, in the information image 303, the plurality of high-luminance pixel groups numbered 1 to 3 in the drawings are distributed within the contour of the cell nucleus region 304.

For the information image 303 as well, the information generating section 182 calculates the total value of the areas of the high-luminance pixel groups 1 to 3 and calculates the percentage of the entire area of the cell nucleus region 304 occupied by this total value. In the example of the drawing, in the cell nucleus with a size corresponding to 578 pixels, the high-luminance pixel groups occupied 18 pixels, which means that the high-luminance pixel groups occupy 3.1% of the total area of the cell nucleus region 304. The calculated value is transferred to the determining section 183 as the information obtained by quantifying the uniformity of the proteins in the sample 101.

After accumulating information obtained by quantifying the uniformity of the protein distribution with the method described above for samples 101 of a plurality of cell sheets cultured simultaneously, the living/dead state of each sample 101 was examined using another method. As a result, among the samples 101 examined, if the percentage of the area of the high-luminance pixel groups was calculated to be less than or equal to 2.5%, it was judged that the cell was living and the proteins were dispersed and distributed inside the cell nucleus region 302. Furthermore, if the percentage of the area of the high-luminance pixel groups was calculated to be greater than 2.5%, it was judged that the cell was dead and the proteins were condensed inside the cell nucleus region 302. Accordingly, by setting a threshold value of 2.5% and comparing the calculated area percentage to this threshold value, the determining section 183 in the determination device 100 can determine that the cell serving as the determination target is dead if the calculated value exceeds this threshold value and that the cell serving as the determination target is alive if the calculated value does not exceed this threshold value.

In the examples described above, the percentage of the area of the cell nucleus occupied by the high-luminance pixel groups is used as the information indicating the uniformity of the distributions of proteins. However, the number of high-luminance pixel groups per unit area, the shapes of individual high-luminance pixel groups, or the like may be used as the information indicating uniformity, for example. Furthermore, a plurality of types of information may be combined to determine the state of a cell.

In this way, in a living cell, the numerical value indicating the uniformity of the proteins based on the ratio of the area of the high-luminance pixel groups to the area of the cell nucleus is small. On the other hand, in a dead cell, this numerical value is large. Accordingly, by observing samples 101 whose living/dead state has been judged in advance, it is possible to determine the threshold value to be used when determining whether the cell is living or dead and to automate the determination of the living/dead state for an unknown cell. In this way, it is possible to automate the quality evaluation of a cell sheet or the like.

In the examples described above, the contours of the cell nucleus regions 302 and 304 are detected with radiation light that does not excite the proteins, lipids, and the like inside the sample 101. However, as described above, lipids within the sample 101 may be irradiated with excitation light having a wavelength that causes a nonlinear optical effect, and the contours of the cell nucleus regions 302 and 304 may be detected according to the difference between the Raman scattered light caused by the proteins and the Raman scattered light caused by the lipids.

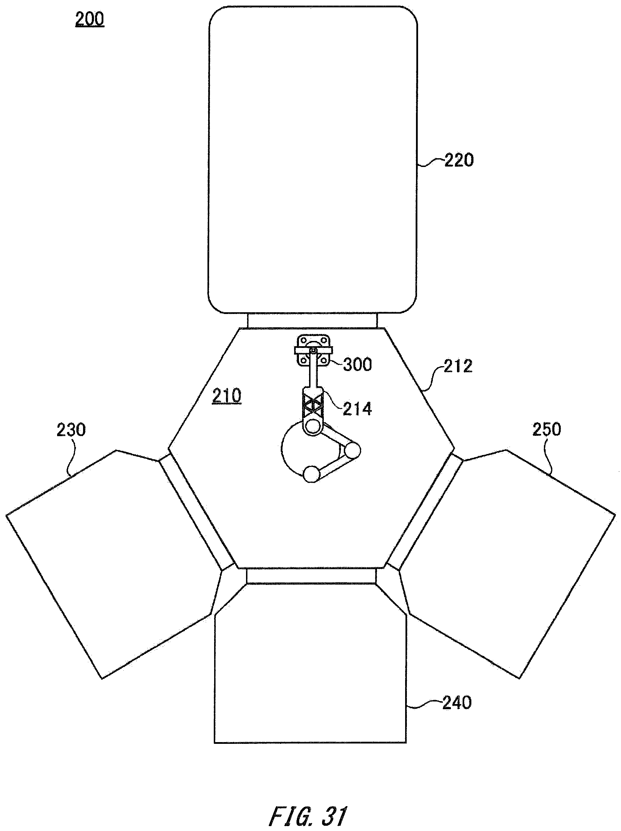

Fig. 31 is a block diagram of a manufacturing device 200 for manufacturing a cell sheet. Specifically, the manufacturing device 200 for manufacturing the cell sheet can be formed incorporating the determination device 100 that performs the series of processes in the determination method described above. The manufacturing device 200 includes a transporting section 210, a loading/unloading section 220, a preparing section 230, a culturing section 240, and a determining section 250.

The transporting section 210 includes an air-tight casing 212 and a transport robot 214 arranged inside the casing 212. The side walls of the casing 212 have a polygonal shape.

The loading/unloading section 220, the preparing section 230, the culturing section 240, and the determining section 250 are coupled in an air-tight manner at respective surfaces of the side walls of the transporting section 210, and the insides of these section are in communication with the inside of the transporting section 210. In this way, the transport robot 214 can transport a culture container 300 without getting contaminated among the loading/unloading section 220, the preparing section 230, the culturing section 240, and the determining section 250.

The loading/unloading section 220 is used when loading materials such as a specimen from which a cell line is to be collected as raw material for the cell sheet, a culture container that has yet to be used, a culture liquid used for culturing, oxygen gas, or the like and when unloading a manufactured cell sheet. In other words, the loading/unloading section 220 blocks the inside of the manufacturing device 200 off from the outside environment, and stops contamination of the materials and cell sheets. The loading/unloading section 220 may be provided with a cleaning apparatus, and may clean the materials loaded into the loading/unloading section 220.

The preparing section 230 includes a device for isolating a cell line to be a cell sheet from the specimen loaded therein. The isolation of the cell line may be realized by a manual operation in a glove box incorporated in the preparing section 230, or may be automated if a large amount of cell sheets are being manufactured.

The culturing section 240 maintains the culture environment of the culture container 300 housing the cell line being cultured. The items maintained as the culture environment can be exemplified by the atmospheric temperature, humidity, carbon dioxide concentration, and the like. Furthermore, the culturing section 240 circulates the culture liquid, oxygen gas, and the like through the culture container, and also includes a waste material and waste liquid tank that collects the culture medium, culture solution, washing solution, and the like used in the culturing process. Furthermore, the culturing section 240 also includes a camera for observing the culturing state of the cell sheet from outside the manufacturing device 200.

The determining section 250 includes the determination device 100 shown in Fig. 1, and also includes a manipulator for mounting the culture container 300 in the determination device 100 and a remote manipulating section for manipulating the determination device 100 from outside the manufacturing device 200.

Other components can also be coupled to the transporting section 210 of the manufacturing device 200. For example, by coupling a layering section that layers a plurality of cultured cell sheets to manufacture a multilayer cell sheet to the transporting section 210, it is possible to perform the entire production process from cultivating the cell sheets to manufacturing the cell sheets without removing the cell sheets from the manufacturing device 200.

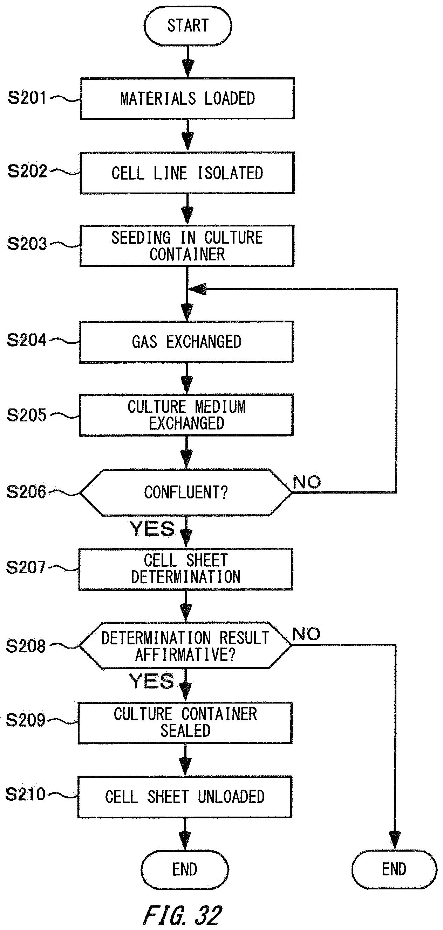

Fig. 32 is a flow chart showing a procedure for manufacturing a cell sheet using the manufacturing device 200 described above. When operating the manufacturing device 200, the operation is performed after air-tight coupling of the transporting section 210, the loading/unloading section 220, the preparing section 230, the culturing section 240, and the determining section 250 to keep the inside sterile.

When manufacturing a cell sheet, first, the materials such as the culture container, the culture medium, the specimen, the culture liquid are prepared, and loaded into the manufacturing device 200 from the loading/unloading section 220 (step S201). Next, the loaded specimen is transported to the preparing section 230 by the transporting section 210, and the cell line that will become the cell sheet is isolated from the loaded specimen (step S202).

Next, the culture medium for culturing is added to the isolated cell line to manufacture a cell suspension with a suitable cell concentration, and this cell suspension is seeded in the culture container 300 (step S203). At this stage, the state of the cells in the cell suspension is determined, and specimens that contain a large amount of dead cells and have a low outlook for being cultured may be removed, for example.

Next, the manufactured culture specimen is transported to the culturing section 240 by the transporting section 210, and the culturing of the cell sheet begins. In the culturing section 240, the atmospheric temperature, humidity, and the like are maintained within a predetermined range, and gas is continually exchanged inside of the culture container 300 to maintain the oxygen concentration, carbon dioxide concentration, and the like of the culture environment to be within a predetermined range (step S204). Furthermore, the culture medium of the culture specimen is periodically exchanged to encourage culturing of the cell sheet (step S205).

Furthermore, while culturing the culture specimen, an investigation is periodically performed to check whether the culture container 300 has become confluent (step S206). If the culture container 300 is not confluent (step S206: NO), the culturing continues until confluency is realized.

On the other hand, the culture container 300 that has become confluent ends the culturing and is transported to the determining section 250 by the transporting section 210, and the quality of the cultured cell sheet is determined (step S207). The cells that have become confluent have an extremely low growing ability, and therefore in the case of a subculture, the culture container 300 is preferably unloaded from the culturing section 240 before becoming confluent.

If the result of the determination of the cultured specimen is that the state of the culture specimen is lower than a predetermined quality (step S208: NO), this specimen is disposed of without being used as a cell sheet. The quality of a cell sheet can be judged using the determination device 100 provided to the determining section 250, for example. The quality determination may be an evaluation based on the number or percentage of living cells or dead cells contained in this cell sheet, for example. Furthermore, the number or percentage of cells that are not dead at the determination stage but are dying can also be one indicator for determining quality of the cell sheet.

If the result of the determination of the cultured specimen is that the state of the culture specimen is higher than a predetermined quality (step S208: YES), the culture container 300 housing this specimen is sealed (step S209) and unloaded from the manufacturing device 200 through the transporting section 210 and the loading/unloading section 220 (step S210). The quality of a cell sheet is determined based on there being a large number of dead cells contained in the cultured cell sheet, for example.

As described above, by manufacturing a cell sheet using the manufacturing device 200, it is possible to realize production from the loading of the materials until the unloading of the sealed cell sheet, without exposing the cell sheet to the outside environment. Accordingly, it is possible to prevent contamination of the cell sheet from the outside and also contamination of workers due to the cell sheet.

Furthermore, high quality cell sheets contain a small number of dead cells or a large number of living cells can be manufactured efficiently. In the manufacturing device 200 described above, the determination for the culture specimen is not limited to being performed around the culturing of steps S204 to S206, and determinations may be repeated at each stage during the culturing. In this way, when the quality of a culture specimen is judged to be worsening during the culturing, this culture specimen can be discarded and hardware resources of the manufacturing device 200 can be transferred to the culturing of another cell sheet.

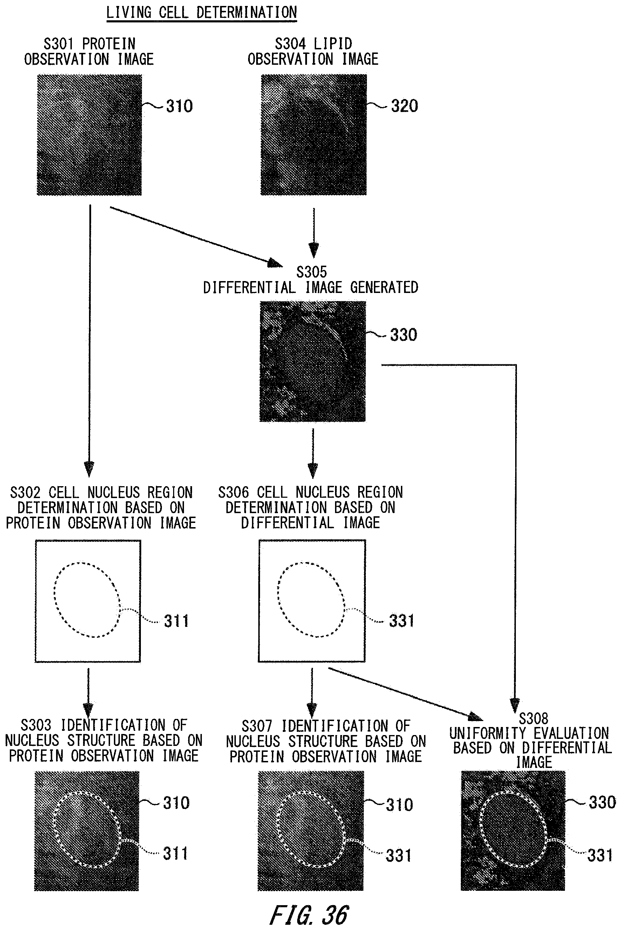

Fig. 36 is a schematic view of the process for determining the state of a cell using Raman scattered light generated by a nonlinear optical effect such as the CARS process. The procedures shown in Fig. 36 correspond to steps S108 to S109 in Fig. 1. Furthermore, Fig. 36 shows the content of each procedure for determining the living/dead state of a cell included in the sample 101, using an image obtained from Raman scattered light.

When determining the state of a cell with the procedures shown in the drawing, first, the protein observation image 310 is formed by generating Raman scattered light caused by proteins as a result of the CARS process with proteins as the target, for example (step S301). By performing image processing such as a binarization process on this observation image, it is possible to directly detect the cell nucleus region 311 occupied by the cell nucleus in the protein observation image 310 (step S302).

In each cell in the sample 101, the proteins are localized inside the nucleus cell, and the amount of lipids is extremely low compared to the amount of proteins. Accordingly, the non-resonant background signal forming a portion of the protein observation image 310 obtained from the Raman scattered light generated by the CARS process with proteins as the target can be treated as information derived from the proteins and not as noise, in at least the cell nucleus region 311.

Then, it is possible to detect the nucleolus, which is one structure in the nucleus also formed by proteins, in the protein observation image 310 shown in Fig. 36, for example. The nucleolus appears white in the cell nucleus region 311 in the drawing. Accordingly, by detecting at least one of the occupancy percentage, area, size, and shape of the nucleolus within the nucleus in the cell nucleus region 311 and comparing this to a predetermined threshold value, it is possible to determine the state of this cell (step S303). The threshold value is determined in advance based on values obtained from samples 101 judged to be living or dead according to staining by the staining reagent. In this way, it is possible to determine the living/dead state of a cell in a small number of steps from step S301 to step S303. This is particularly effective when the acquired protein observation image 310 is high quality.

On the other hand, if it is difficult to detect the cell nucleus region 311 from the protein observation image 310, following step S301, the lipid observation image 320 is formed by generating Raman scattered light caused by lipids through the CARS process with lipids as the target, for example (step S304). Furthermore, a differential image 330 is generated based on the difference between the acquired lipid observation image 320 and the protein observation image 310 acquired at step S301 (step S305).

In a cell, proteins and lipids are present in an exclusive manner. Accordingly, it is possible to detect the cell nucleus region 331 from which the effect of the non-resonant background signal has been eliminated, from the differential image 330 described above (step S306). Next, by identifying the cell nucleus region 331 detected from the differential image 330 in the protein observation image 310 described above, it is possible to identify the state of the cell in the sample 101 based on the protein observation image 310 (step S307).

With the cell nucleus region 331 detected at step S306, the state of the cell in the sample 101 may be determined based on the uniformity of the proteins in the cell nucleus region 331 of the differential image 330 itself. In this way, the determination procedure may be adjusted according to the state of the sample 101, the capabilities of the microscope, or the like, although this does increase the number of steps before the determination.

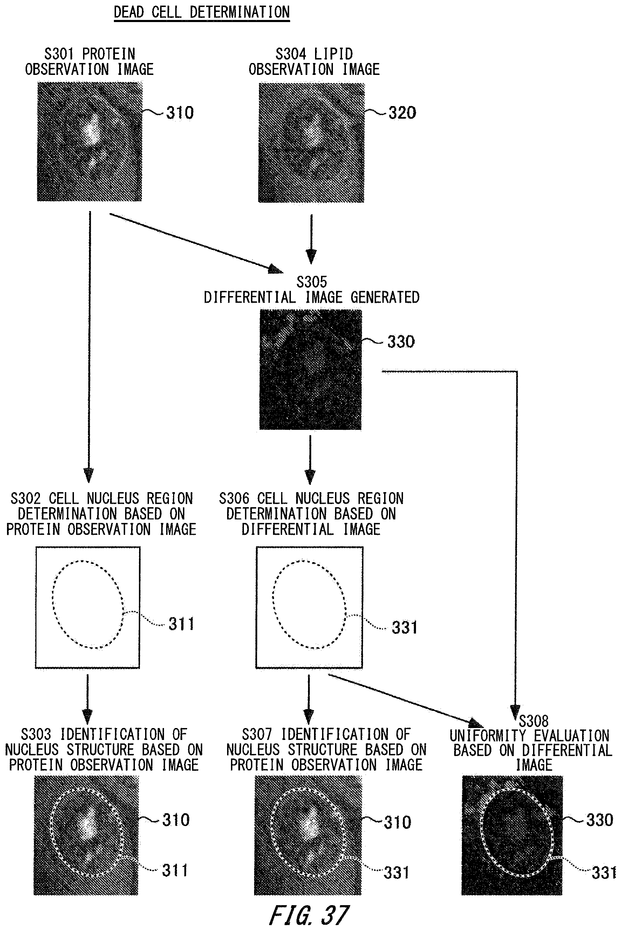

Fig. 36 shows an example in which the state is determined for a living cell, i.e. a cell in which the numerical value indicating the uniformity of the proteins in the cell nucleus is less than the threshold value in the embodiment described above. Fig. 37 shows an example of a case in which a state determination of the sample 101 according to the same procedures as shown in Fig. 36 is applied to a sample 101 including dead cells, i.e. cells in which the numerical value indicating the uniformity is greater than the threshold value. The applied procedures are the same as those shown in Fig. 36, but at steps S303 and S307, the shape, size, and the like of the nucleolus in the protein observation image 310 and the cell nucleus regions 311 and 331 are judged to be significantly different from those of the nucleolus of the living cell shown in the protein observation image 310 of Fig. 36. Furthermore, in the differential image 330 at step S408 as well, uniformity of the proteins breaks down, and is judged to be skewed near the center of the cell nucleus region 331.

In the embodiments described above, examples are shown in which the cell type serving as the investigation target is a myoblast, but a type of cell other than a myoblast may be the investigation target. In this case, the threshold value used for the state determination of the cell is set according to each type of cell.

Furthermore, if the cause of death of a cell is apoptosis, there are cases where proteins appear on the surfaces of the cell membranes without the cell membranes being damaged at the initial stage immediately after death. In such a case, the proteins appearing on the surface of the cell membrane can be stained by the staining reagent, and therefore it is possible to judge the state of the cell based on the state of the proteins on the surface of the cell membrane by storing in advance a correspondence between the distribution of proteins within the cell nucleus and the distribution of proteins on the surface. In this case, the initial stage is also included in the state where the cell is dead.

Furthermore, in the embodiments described above, examples are shown in which the state of a cell is determined based on the uniformity of the proteins in a two-dimensional space, but as another example, a two-dimensional image may be acquired at positions at a plurality of heights for a single cell nucleus, the three-dimensional uniformity of the proteins in the cell nucleus may be detected based on these images, and the state of the cell may be determined based on the detected uniformity.

While the embodiments of the present invention have been described, the technical scope of the invention is not limited to the above described embodiments. It is apparent to persons skilled in the art that various alterations and improvements can be added to the above-described embodiments. It is also apparent from the scope of the claims that the embodiments added with such alterations or improvements can be included in the technical scope of the invention.

The operations, procedures, steps, and stages of each process performed by an apparatus, system, program, and method shown in the claims, embodiments, or diagrams can be performed in any order as long as the order is not indicated by "prior to," "before," or the like and as long as the output from a previous process is not used in a later process. Even if the process flow is described using phrases such as "first" or "next" in the claims, embodiments, or diagrams, it does not necessarily mean that the process must be performed in this order.

### List of Reference Numerals

100: determination device, 101: sample, 102: slide glass, 103: spacer, 104: cover glass, 105: sealing material, 106: sealing liquid, 109: cell sheet, 110: stage, 111: stage scanner, 120: objective optical system, 121: upper objective lens, 122: lower objective lens, 130: laser device, 131, 132: laser light source, 133: combiner, 140: culturing section, 141: galvanic scanner, 142: scanner lens, 143: primary image surface, 144, 151: reflective mirror, 150: upper Raman scattered light detecting section, 152, 153, 162, 163: relay lens, band-pass filter 154, 164: band-pass filter, 155, 165: photoelectrical intensifier tube, 160: lower Raman scattered light detecting section, 161: dichroic mirror, 170: control section, 171: processing apparatus, 172: mouse, 173: keyboard, 174: display section, 181: detecting section, 182: information generating section, 183: determining section, 200: manufacturing device, 210: transporting section, 212: casing, 214: transfer robot, 220: loading/unloading section, 230: preparing section, 240: culturing section, 250: determining section, 300: culture container, 301, 303: information image, 302, 304, 311, 331: cell nucleus region, 310: protein observation image, 320: lipid observation image, 330: differential image, 401, 402: optical micrograph

## Claims

1. A determination device comprising:
a determining section that determines a state of a cell, using information relating to uniformity of a detection target generated based on an optical intensity of radiation light from the detection target included in a biological cell irradiated with excitation light.

2. The determination device according to Claim 1, wherein
the detection target is a protein.

3. The determination device according to Claim 1 or 2, comprising:
an information generating section that generates the information, wherein
the information generating section generates the information from which information corresponding to a non-resonant background signal is excluded.

4. The determination device according to Claim 3, wherein
the information generating section generates the information based on differences between the optical intensity of the detection target and the optical intensity of another detection target contained in the biological cell, at a plurality of positions in the biological cell.

5. The determination device according to Claim 4, wherein
the other detection target is a lipid.

6. The determination device according to Claim 4, wherein
the information generating section further generates information indicating a range in which the radiation light is detected, based on an image reflecting a refractive index distribution in the biological cell including the detection target.

7. The determination device according to any one of Claims 3 to 6, wherein
the determining section determines the state based on a threshold value obtained based on a determination result of a cell whose state has already been known.

8. The determination device according to any one of Claims 3 to 7, wherein
the information generating section generates the information as a numerical value, and
the determining section determines the state based on a threshold value defined by a numerical value.

9. The determination device according to Claim 7 or 8, wherein
the information generating section generates an intensity ratio of a maximum intensity of the radiation light to an average intensity of the radiation light, and
the determining section determines the state based on the threshold value relating to the intensity ratio.

10. The determination device according to any one of Claims 7 to 9, wherein
the information generating section identifies a Gaussian function obtained by performing Gaussian fitting on a histogram of the optical intensity and generates an integrated value of a frequency up to the optical intensity for which the Gaussian function has a predetermined slope, and
the determining section determines the state based on the threshold value relating to the integrated value.

11. The determination device according to any one of Claims 7 to 10, wherein
the information generating section identifies a Gaussian function obtained by performing Gaussian fitting on a histogram of the optical intensity and generates a difference between the optical intensity at a peak position of the identified Gaussian function and an average value of the optical intensity, and
the determining section determines the state based on the threshold value relating to the difference.

12. The determination device according to any one of Claims 7 to 11, wherein
the information generating section generates a contrast evaluation value for the optical intensity, and
the determining section determines the state based on the threshold value relating to the contrast evaluation value.

13. The determination device according to Claim 12, wherein
the information generating section sets the contrast evaluation value in one region to be an average value of a ratio of a minimum value to a maximum value of the optical intensity of the radiation light in the one region of the biological cell and a ratio of a minimum value to a maximum value of the optical intensity of the radiation light in a neighboring region of the one region.

14. The determination device according to any one of Claims 7 to 13, wherein
the information generating section generates a Lorentz curve obtained by integrating a frequency of the optical intensity and generates an area difference between the Lorentz curve and a line of perfect equality, and
the determining section determines the state based on the threshold value relating to the area difference.

15. The determination device according to Claim 14, wherein
the information generating section generates the Lorentz curve in which the optical intensity is normalized.

16. The determination device according to any one of Claims 7 to 15, wherein
the information generating section performs a Fourier transform on a predetermined region of a two-dimensional image formed based on a distribution of the optical intensity and generates a ratio of a predetermined frequency component, and
the determining section determines the state based on the threshold value relating to the ratio.

17. The determination device according to any one of Claims 7 to 16, wherein
the information generating section generates the uniformity that has been quantified by replacing each optical intensity associated with a position in space with a weighted average of the optical intensity in a predetermine region including the position, and
the determining section determines the state based on the threshold value relating to the uniformity; and

18. The determination device according to any one of Claims 7 to 17, wherein
the information generating section generates an area ratio of a high-luminance region detected corresponding to the detection target present in clumps in the biological cell, and
the determining section determines the state based on the threshold value relating to the area ratio.

19. The determination device according to Claim 18, wherein
the information generating section sets the area ratio to be a ratio of a region in which high-luminance pixels exist continuously in a binary image obtained by binarizing, in a range of two times a standard deviation σ, a two-dimensional image formed based on a distribution of the optical intensity.

20. The determination device according to any one of Claims 1 to 19, wherein
the radiation light is anti-Stokes Raman scattered light radiated from the detection target.

21. The determination device according to any one of Claims 1 to 20, comprising:
a detecting section that detects the optical intensity, wherein
the detecting section detects the optical intensity of radiation light obtained non-invasively for the cell.

22. A determination program that causes a processor to:
determine a state of a cell using information relating to uniformity of a detection target generated based on an optical intensity of radiation light from the detection target included in a biological cell irradiated with excitation light.

23. A determination method comprising:
determining a state of a cell using information relating to uniformity of a detection target generated based on an optical intensity of radiation light from the detection target included in a biological cell irradiated with excitation light.

24. A cell sheet manufacturing device comprising:
a preparing section that prepares a cell line by isolating a cell;
a culturing section that cultures the cell line in a cell sheet; and
the determination device according to any one of Claims 1 to 21.

25. The cell sheet manufacturing device according to Claim 24, wherein
the determination device determines pass/fail of the cell sheet.

26. A cell sheet manufacturing method comprising:
preparing a cell line by isolating a cell;
culturing the cell line in a cell sheet; and
determining a state of the cell using the determination device according to any one of Claims 1 to 21.

27. The cell sheet manufacturing method according to Claim 26, wherein
the determining includes determining pass/fail of the cell sheet.
